(19) Europäisches Patentamt / European Patent Office / Office européen des brevets

(11) **EP 2 302 362 B1**

(12) **FASCICULE DE BREVET EUROPEEN**

(45) Date de publication et mention
de la délivrance du brevet:
**02.10.2019 Bulletin 2019/40**

(51) Int Cl.:
*G01N 21/47* *(2006.01)*          *G01N 21/64* *(2006.01)*
*A61B 5/00* *(2006.01)*          *G06T 11/00* *(2006.01)*

(21) Numéro de dépôt: **10179184.6**

(22) Date de dépôt: **24.09.2010**

(54) **Dispositif et procédé de reconstruction spatiale d'une cartographie de fluorescence**

Vorrichtung und Verfahren zur räumlichen Abbildung einer Fluoreszenz-Kartenaufnahme

Device and method for spatial reconstruction of a fluorescence cartography

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **24.09.2009 FR 0956610**

(43) Date de publication de la demande:
**30.03.2011 Bulletin 2011/13**

(73) Titulaire: **Commissariat à l'Énergie Atomique
et aux Énergies Alternatives
75015 Paris (FR)**

(72) Inventeurs:
- **HERVE, Lionel
  38700 Corenc (FR)**
- **DINTEN, Jean-Marc
  69008, LYON (FR)**
- **LECORDIER, Ludovic
  14400, AGY (FR)**

(74) Mandataire: **Ilgart, Jean-Christophe
BREVALEX
95, rue d'Amsterdam
75378 Paris Cedex 8 (FR)**

(56) Documents cités:
EP-A1- 1 884 765          WO-A1-2006/032151
WO-A2-02/41760          WO-A2-2007/080326
US-A1- 2002 072 677

- HE H ET AL: "An analytic reflection method for time-domain fluorescence diffuse optical tomography based on a generalized pulse spectrum technique" PROGRESS IN BIOMEDICAL OPTICS AND IMAGING - PROCEEDINGS OF SPIE - MULTIMODAL BIOMEDICAL IMAGING III, vol. 6850, 2008, XP002579460 SPIE US DOI: 10.1117/12.756377
- GUYON L ET AL: "Time-resolved fluorescence tomography in cancer research: backward versus toward geometry" PROCEEDINGS OF THE SPIE ON OPTICAL TOMOGRAPHY AND SPECTROSCOPY OF TISSUE VIII, vol. 7174, 12 février 2009 (2009-02-12), XP002579495 THE INTERNATIONAL SOCIETY FOR OPTICAL ENGINEERING USA ISSN: 0277-786X DOI: 10.1117/12.808237
- L. HERVE' ET AL.: "Localization of fluorescence marked prostate tumor with time-resolved diffuse optical tomography" MULTIMODAL BIOMEDICAL IMAGING, V ,PROCEEDINGS OF SPIE, vol. 7557, 75570F, 23 février 2010 (2010-02-23), pages 1-9, XP002617075

**Description**

**DOMAINE TECHNIQUE ET ART ANTÉRIEUR**

L'invention concerne le domaine de l'imagerie moléculaire de fluorescence sur les tissus biologiques par des méthodes optiques résolues en temps.

Elle s'applique notamment à l'imagerie moléculaire optique sur le petit animal et à l'imagerie moléculaire optique sur l'homme (cerveau, sein, autres organes où des fluorophores peuvent être injectés).

L'application particulièrement visée est la détection de cancer de la prostate.

A l'heure actuelle, la localisation de cellules malades peut se faire à l'aide des techniques de biopsie.

Mais, afin de trouver la position, même approximative, de ces cellules, on doit effectuer plusieurs prélèvements. Cette technique invasive est évidemment délicate à mettre en oeuvre et est consommatrice de temps.

Il se pose donc le problème de la détermination de la localisation, même de manière approximative, de fluorophores fixés sur une zone d'un milieu, par exemple un milieu biologique.

Dans certains cas, on cherche même à localiser des fluorophore en vue d'une intervention chirurgicale ultérieure.

Les techniques optiques d'imagerie moléculaire de fluorescence se développent donc de plus en plus actuellement grâce à l'utilisation de marqueurs fluorescents spécifiques. Ceux-ci viennent se fixer de façon préférentielle sur les cellules cibles d'intérêt (par exemple des cellules cancéreuses) et offrent un meilleur contraste de détection que les marqueurs non spécifiques. Ces techniques ont pour but de localiser spatialement la fluorescence mais aussi d'en déterminer la concentration.

Les systèmes de tomographie optique utilisent diverses sources de lumière. Il existe donc des appareils en mode continu, des appareils en mode fréquentiel (qui utilisent des lasers modulés en fréquence) et enfin des appareils fonctionnant en mode temporel, qui utilisent des sources lumineuses pulsées, ou sources lumineuses impulsionnelles, telles des Lasers pulsés.

Les données temporelles sont obtenues lorsqu'on utilise une source de rayonnement impulsionnelle, ou source pulsée, délivrant un signal bref à une cadence déterminé. On parle alors d'imagerie diffuse résolue en temps. Les données temporelles sont celles qui contiennent le plus de contenu informationnel sur le tissu traversé, mais pour lesquelles les techniques de reconstruction sont les plus complexes. La mesure en chaque point d'acquisition est en effet une fonction dépendante du temps (appelée TPSF pour « Temporal Spread Function », ou fonction d'étalement temporel).

On cherche à extraire des paramètres simples de la TPSF, dont on connaît par ailleurs l'expression théorique. Ensuite, la résolution du problème inverse permet de retrouver la distribution de fluorescence.

On connaît des techniques de localisation, par exemple du document EP 1884765.

Ce document montre un procédé où un fluorophore unique est localisé en minimisant un paramètre obtenu par la somme, pour les diverses positions relatives fibres-fluorophore, des différences entre la mesure, pour chaque position, du moment d'ordre 1 de la distribution temporelle de fluorescence, ou temps de vol, et une expression analytique théorique de ce temps de vol.

Le document WO2006/32151 divulgue un procédé pour déterminer la concentration d'un fluorophore en fonction des coordonnées spatiales, basé sur un moment d'ordre donné de la distribution temporelle du signal de fluorescence (TPFS).

Mais ces techniques ne donnent pas satisfaction, en particulier dans la géométrie dite en « rétrodiffusion » où sources et détecteurs sont d'un même coté de l'objet ou du milieu étudié.

**EXPOSÉ DE L'INVENTION**

Il est apparu aux inventeurs que, lors de l'application des techniques connues d'imagerie optique diffuse résolue en temps, un certain nombre de problèmes se posent. En effet, différentes répartitions de fluorophores peuvent aboutir à des mêmes mesures, une mesure étant par exemple l'intensité de fluorescence ou le temps de vol moyen, notamment en géométrie de réflexion.

Pour un milieu quelconque, connaissant ses propriétés optiques, on peut calculer la densité de photons pour une source de Dirac, ponctuelle et localisée en $r_s$ et à t=0s, à l'aide des fonctions de Green.

Ces fonctions $G_s(r,t)$ peuvent être déterminées en utilisant une simulation Monte-Carlo, ou en résolvant l'équation de transfert radiatif ou, le plus souvent, en résolvant l'équation de diffusion (1) auquel on ajoute des conditions de bord :

$$\frac{\partial G_s(r,t)}{c\partial t} - \nabla D \nabla G_s(r,t) + \mu_a G_s(r,t) = \delta(r - r_s, t) \tag{1}$$

Les moments de Gs, c'est-à-dire les quantités :

$$G_s^{(n)}(r) = M^n(G_s) = \int_0^\infty Gs(r,t)t^n dt \quad (1')$$

suivent les équations différentielles données par (2) :

$$-\nabla D\nabla G_s^{(n)}(r) + \mu_a G_s^{(n)}(r) = \delta(r - r_s, n) + \frac{n}{c} G_s^{(n-1)} \quad (2)$$

Ainsi, $G_s^{(0)}$ est la solution de l'équation différentielle (2) avec seulement le Dirac comme terme source, et $G_s^{(n)}$ est la solution lorsque le terme source est proportionnel à $G_s^{(n-1)}$. La suite des fonctions $G_s^{(n)}$ se trouve donc par étapes successives.

Dans le cas d'un milieu homogène infini, une solution analytique de (1) est connue. Il s'agit de :

$$G_s^\infty(r,t) = \frac{c}{(4\pi Dct)^{3/2}} \exp(-\frac{r^2}{4Dct} - \mu_a ct) \quad (3)$$

Les moments de cette fonction sont déterminés aussi analytiquement.

Les 2 premiers sont :

$$G_s^{(0)}(r) = \exp(-k.|r - r_s|)/(|r - r_s|D) \quad (4)$$

$$G_s^{(1)}(r) = \exp(-k.|r - r_s|)/(2c\sqrt{\mu_a D}) \quad (5)$$

Ces formules sont intéressantes pour simuler des mesures pour lesquelles on est capable d'insérer les sources (et détecteurs) dans le milieu (par exemple grâce à des fibres optiques au bout d'une aiguille de biopsie).

Dans la suite de la demande, certains modes de réalisation feront appel aux moments 0 et 1 de la mesure $M_{sd}(t)$ provoquée par la source s et mesurée par le détecteur d.

Ils sont utiles car ils donnent l'énergie totale détectée $I_{sd}$ et le temps de vol $T_{sd}$.

En effet :

$$I_{sd} = \int_0^\infty M_{sd}(t).t^0 dt = M_{sd}^{(0)} \quad (6)$$

et

$$T_{sd} = \frac{\int_0^\infty M_{sd}(t).t^1.dt}{\int_0^\infty M_{sd}(t).t^0.dt} = M_{sd}^{(1)} / M_{sd}^{(0)} \quad (7)$$

La description de la chaîne des processus physiques est la suivante ; chaque photon est :

- émis par la source suivant une densité de probabilité décrite par la réponse temporelle de la source,
- puis se propage suivant la probabilité décrite par la fonction $G_s(r,t)$ jusqu'à un point r du maillage,

- puis est absorbé suivant la concentration et la section efficace du fluorophore en r,
- puis est émis par le fluorophore à la longueur d'onde de fluorescence en r,
- puis se propage jusqu'au détecteur d suivant une probabilité $G_d(r,t)$
- et enfin est détecté par le détecteur, ce dernier ayant une réponse temporelle.

Du fait des propriétés de retour inverse de la lumière, $G_d(r,t)$ est la solution de (1) en remplaçant $r_s$ par $r_d$ ($r_d$ étant la position du détecteur considéré).

Au final, pour un fluorophore « Dirac » disposé en r, la mesure $M_{sd}$ effectuée par le détecteur d pour un milieu illuminé par la source s s'écrit :

$$M_{sd}(t) = S(t) * G_s(r,t) * F(r,t) * G_d(r,t) * D(t) \quad (8)$$

Le signe * représente la convolution temporelle, $F(t)$ est la réponse temporelle du fluorophore, $S(t)$ est la fonction temporelle de la source et $D(t)$ est la réponse temporelle du détecteur.

En pratique, on est capable de déterminer le signal source détecté par le détecteur, ce qui permet d'aboutir à une convolution des réponses temporelles respectives de la source et du détecteur. Autrement dit, il n'est pas nécessaire d'estimer séparément la réponse temporelle de la source et la réponse temporelle du détecteur, c'est-à-dire la convolution $S(t) * D(t)$.

Ici, nous avons supposé que les fonctions temporelles des sources et des détecteurs sont indépendantes de leur position ; toutefois, si ce n'était pas le cas, on pourrait aisément traiter ce problème en écrivant $S_s$ et $D_d$, les indices s et d étant les indices des différentes sources et des différents détecteurs.

Pour une assemblée de fluorophores dans le volume $\Omega$, nous obtenons :

$$M_{sd}(t) = \iiint_\Omega S(t) * G_s(r,t) * F(r,t) * G_d(r,t) * D(t) dr \quad (9)$$

Du fait des propriétés de calcul des moments sur les produits de convolutions, nous obtenons pour les deux premiers moments de Msd(t) :

$$M_{sd}^{(0)} = \iiint_\Omega S^{(0)}.G_s^{(0)}(r).F^{(0)}(r).G_d^{(0)}(r).D^{(0)} dr \quad (10)$$

$$M_{sd}^{(1)} = \iiint_\Omega dr \begin{cases} S^{(1)}.G_s^{(0)}(r).F^{(0)}(r).G_d^{(0)}(r).D^{(0)} + \\ S^{(0)}.G_s^{(1)}(r).F^{(0)}(r).G_d^{(0)}(r).D^{(0)} + \\ S^{(0)}.G_s^{(0)}(r).F^{(1)}(r).G_d^{(0)}(r).D^{(0)} + \\ S^{(0)}.G_s^{(0)}(r).F^{(0)}(r).G_d^{(1)}(r).D^{(0)} + \\ S^{(0)}.G_s^{(0)}(r).F^{(0)}(r).G_d^{(0)}(r).D^{(1)} \end{cases} \quad (11)$$

En s'inspirant de (7) nous obtenons :

$$M_{sd}^{(1)} = \iiint_\Omega dr S^{(0)}.G_s^{(0)}(r).F^{(0)}(r).G_d^{(0)}(r).D^{(0)}(T_s + T_s(r) + T(r) + T_d(r) + T_d) \quad (12)$$

Dans cette formule :

- $T_s$ est le temps moyen de la fonction source,
- $T_s(r)$ est le temps de vol moyen entre s et r,
- $T(r)$ est le temps de vie $\tau$ du fluorophore, censé être invariant suivant la position,
- $T_d(r)$ est le temps de vol moyen entre r et le détecteur d,

- $T_d$ est le temps de réponse moyen du détecteur.

Dans le cas homogène infini, on voit d'après les formules (4) et (5) que Ts(r), temps de vol moyen entre s et r est juste égal à :

$$\left|r - r_s\right|/(2c\sqrt{\mu_a D})$$.

Ainsi, $v = 2c\sqrt{\mu_a D}$ s'interprète comme la vitesse moyenne des photons dans ce milieu.

Dans le cas de la prostate, on obtient typiquement v=5cm/ns. De même pour Td(r).

Lorsque le fluorophore est unique et ponctuel, les formules (10) et (12) se simplifient.

Si le fluorophore se trouve à la position $r_0$, les formules deviennent :

$$I_{sd} = M_{sd}^{(0)} = S^{(0)}.G_s^{(0)}(r_0).F^{(0)}(r_0).G_d^{(0)}(r_0).D^{(0)} \quad (13)$$

et

$$T_{sd} = \frac{M_{sd}^{(1)}}{M_{sd}^{(0)}} = T_s + T_s(r_0) + \tau + T_d(r_0) + T_d \quad (14)$$

$s^{(0)}D^{(0)}$ et Ts + Td peuvent être mesurés au cours d'une opération de calibration, au cours de laquelle la source est placée face au détecteur. On obtient alors un signal de calibration, dont le moment d'ordre 0 est égal à $S^{(0)}D^{(0)}$ et le moment normé d'ordre 1 est égal à Ts+Td

Ainsi, le temps de vol mesuré est égal au retard moyen de la source auquel s'ajoutent le temps de vol entre la source et le fluorophore, le temps de vie du fluorophore, le temps de vol entre le fluorophore et le détecteur et le temps de réponse du détecteur.

Passons maintenant au problème de la reconstruction de la fluorescence, d'abord dans le cas d'une assemblée de fluorophore. Or on constate que, en rétrodiffusion, la reconstruction des cartes de fluorescence dans un milieu diffus est très difficile.

Les inventeurs ont constaté que, notamment en géométrie de réflexion, différentes configurations aboutissent à des mesures identiques. Ainsi, un fluorophore réparti de façon étendue peut produire une mesure équivalente à un flurophore ponctuel. Il apparaît donc que, lors de la reconstruction de fluorophores en réflexion, on a un fort besoin d'hypothèses a priori. Un type d'hypothèse que l'on peut utiliser est de dire que les fluorophores sont ponctuels et localisés en des lieux donné. Une telle hypothèse peut s'appeler une contrainte de support.

Une première solution est de considérer que le fluorphore est unique et localisé de façon ponctuelle dans le milieu étudié.

Une autre solution est de considérer une pluralité de fluophores ponctuels répartis dans le milieu étudié.

La formule (14) ci-dessus permet de reconstruire la fluorescence par « triangulation » , par exemple à l'aide de la technique décrite dans le document EP 1884765.

En particulier, lorsque le milieu est homogène infini :

$$T_{sd} - T_s - \tau - T_d = \left(\left|r - r_s\right| + \left|r - r_d\right|\right)/(2c\sqrt{\mu_a D})$$.

Ainsi, en combinant plusieurs couples source-détecteur, on peut remonter à la position r. L'inconvénient de cette méthode est qu'elle fournit autant de solutions qu'il y a de couples source-détecteur.

L'invention propose à cette fin un procédé de localisation d'au moins un fluorophore, selon la revendication 1 .

L'invention concerne également un dispositif de localisation d'au moins un fluorophore, selon la revendication 6

Dans un tel dispositif les sources de rayonnement et les détecteurs sont de préférence en géométrie de réflexion.

Dans un procédé selon l'invention, on peut réaliser, en outre, pour localiser plusieurs fluorophores, une étape d'ajustement, à l'intensité mesurée, de l'ensemble des contributions à l'intensité des fluorophores déjà localisés.

Un dispositif selon l'invention peut comporter des moyens pour réaliser, dans le cas d'une pluralité de fluorophores, une étape d'ajustement, à l'intensité mesurée, de l'ensemble des contributions à l'intensité des fluorophores déjà localisés.

## BRÈVE DESCRIPTION DES DESSINS

- Les figures 1A-1B sont des schémas d'une configuration expérimentale de tomographie de fluorescence dans la géométrie de réflexion, pouvant être mise en oeuvre dans le cadre de l'invention.
- Les figures 1C-1E représentent respectivement une série d'impulsions laser et de photons uniques émis, et une courbe de fluorescence obtenue à partir des données relatives aux photons uniques, ainsi que des courbes de fluorescence mesurées.
- La figure 2 est un schéma d'une configuration de fluorophores, de sources et de détecteurs utilisée pour des tests.
- Les Figures 3A-3C, 4A-4C illustrent l'application de critères non combinés, selon l'invention.
- Les Figures 5A-5B illustrent l'application d'un critère combiné, selon l'invention.
- La Figure 6 est un schéma d'une configuration de 2 fluorophores, de 2 sources et de 2 détecteurs.
- Les Figures 7 et 8 sont des exemples de procédé selon l'invention.
- Les Figures 9A-9C et 10A-10H illustrent des exemples d'application d'un procédé selon l'invention.
- Les Figure 11A-11C et 12A-12C illustrent des résultats obtenus avec un procédé selon l'invention.

## EXPOSÉ DÉTAILLÉ DE MODES DE RÉALISATION DE L'INVENTION

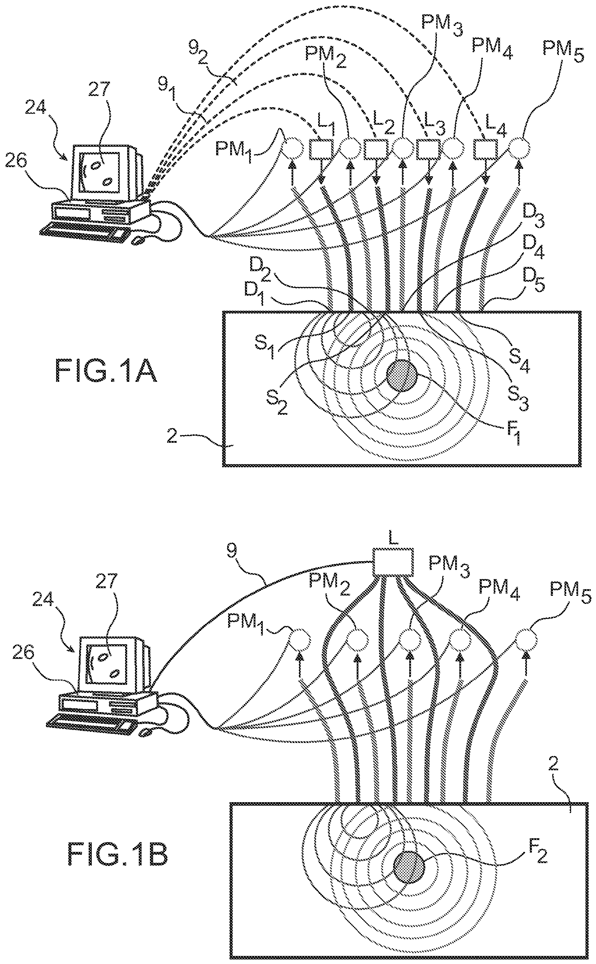

Un dispositif expérimental dans une géométrie en réflexion (où les sources et détecteurs se trouvent sur la même face de l'objet ou du milieu étudié) est représenté en figure 1A.

La source de rayonnement et les moyens de détection peuvent être respectivement l'extrémité d'une fibre optique qui amène la lumière d'excitation dans le milieu et l'extrémité d'une fibre optique qui prélève une partie de la lumière d'émission.

On y distingue le milieu diffusant 2 contenant des inclusions fluorescentes $F_i$ (ici une inclusion $F_1$ est représentée), un ensemble de sources $S_i$ (chacune des sources, par exemple une source laser $L_i$, est ici reliée à une fibre, donc on assimile la source à l'extrémité $S_i$, la plus proche du milieu 2, de la fibre) amenant la lumière séquentiellement dans le milieu.

Un ensemble de points de détection est désigné par $D_i$ : chacun des détecteurs $PM_i$ est ici relié à une fibre, donc on assimile le détecteur à l'extrémité, la plus proche du milieu 2, de la fibre. Ces détecteurs permettent d'échantillonner la densité de photons de fluorescence.

D'une manière générale, le nombre $n_s$ de sources peut être quelconque. Il peut par exemple y avoir une seule source, ou 2 ou 3, ou tout nombre $n_s > 3$.

Le nombre $n_d$ de détecteurs peut être quelconque. Il peut par exemple y avoir un seul détecteur, ou 2 ou 3, ou tout nombre $n_d > 3$.

De préférence, on a $n_s > 2$ ou $> 3$ ou $> 5$ et $n_d > 2$ ou $> 3$ ou $> 5$.

Dans des applications ou une forte compacité est requise, en endoscopie par exemple, le nombre $n_s$ de sources sera compris entre 1 et 10.

Chaque source $L_i$ peut être une source laser impulsionnel ou une fibre optique reliée à un laser impulsionnel déporté (ce qui est le cas de la configuration de la figure 1A). On peut aussi avoir une seule source connectée à plusieurs fibres (configuration de la figure 1B où il y a une source unique laser L, les autres aspects du système étant les mêmes que ceux de la figure 1A) , et des moyens, par exemple un commutateur optique ou un multiplexeur pour sélectionner la fibre dans laquelle le faisceau est envoyé.

Chaque détecteur $D_i$ peut comporter un ensemble des pixels de caméras de type CCD ou CMOS, ou comporter un photomultiplicateur, et il peut être relié à une fibre optique qui transmet le signal du milieu étudié vers le détecteur, ce qui est le cas de la configuration des figures 1A et 1B.

Selon un mode de réalisation, en sortie du ou des détecteurs, on réalise une analyse temporelle des signaux à l'aide d'instrument de mesure dédié (TCSPC Time Correlated Single Photon Couting - dispositif de comptage de photons uniques correlés en temps ou caméra intensifiée avec une porte temporelle).

L'une des sources d'impulsions de rayonnement $L_i$ peut également être utilisée comme moyen de déclenchement de la carte TCSPC (par exemple par une liaison $9_1$, $9_2$, 9 entre chaque source $L_i$ et les moyens 24) .

Il est préférable de travailler avec des impulsions dans le domaine de la femto seconde, à condition de disposer de la source de rayonnement adéquate, c'est-à-dire d'une ou de plusieurs sources laser $L_i$ dont chaque impulsion a une largeur temporelle également dans le domaine de la femto seconde. L'utilisation de sources pulsées du type diode Laser picoseconde est également envisageable, l'avantage d'une telle solution étant un moindre coût.

Selon un mode particulier de réalisation, une ou plusieurs sources $L_i$ est une diode laser pulsée, par exemple à une longueur d'onde voisine de 630 nm et avec un taux de répétition d'environ 50 MHz.

La lumière Laser passe de préférence par un filtre interférentiel pour éliminer d'éventuels modes secondaires. Un filtre interférentiel et/ou un filtre coloré absorbant les faibles longueurs d'onde peuvent être placés devant chaque détecteur $PM_i$ ou l'extrémité $D_i$ de la fibre correspondante pour sélectionner la lumière de fluorescence (par exemple : $\lambda > 650$ nm, la source étant à une longueur d'onde de, par exemple, 631 nm) d'un fluorophore $F_i$ disposé dans le milieu 2 et optimiser l'élimination de la lumière d'excitation.

Dans le texte de la présente demande, quel que soit le mode de réalisation de l'invention, il sera notamment question de la position de la source de rayonnement, et/ou de la position des moyens de détection.

Lorsque des fibres sont utilisées, ces positions s'entendent le plus souvent comme celles des extrémités des fibres qui amènent le rayonnement dans le milieu diffusant 2, ou à l'interface de ce milieu, et /ou celles qui collectent le rayonnement diffusé, ces dernières étant placées dans le milieu ou à l'interface de ce milieu, mais ne s'entendent alors pas comme étant celles de la source $L_i$ proprement dite ou du détecteur $PM_i$ proprement dit. Les fibres amenant le rayonnement dans le milieu peuvent être appelées fibres d'excitation, et les fibres collectant le rayonnement diffusé peuvent être appelées fibres d'emission, ou fibres de collection.

En application de type « endoscopie », on utilise préférentiellement un système à fibres, les fibres pouvant être par exemple intégrées dans une sonde endo-rectale, au moins une fibre transmettant la source de lumière pulsée d'excitation à la zone à examiner.

Cette source de lumière pulsée peut être externe à la sonde.

Au moins une autre fibre transmet le signal optique d'émission de la zone à examiner vers un photodétecteur, qui peut lui aussi être externe à la sonde. Selon la technique TCSPC (pour « Time Correlated Single Photon Counting », ou comptage de photons uniques correlés en temps) on détecte, à l'aide du photomultiplicateur, un photon émis par le fluorophore après une impulsion de la source de rayonnement.

Le système permet donc une détection résolue en temps des impulsions de fluorescence.

Il permet de récupérer des photons de fluorescence.

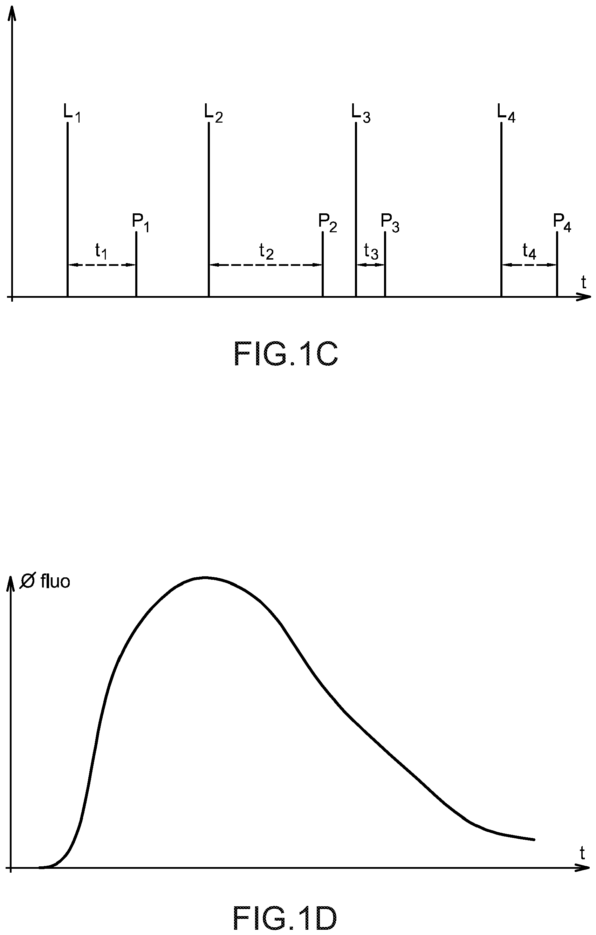

La figure 1C représente une série d'impulsions laser $I_i$ (i = 1 - 4) et une série de photons uniques $p_i$ (i = 1 - 4) correspondants, ces derniers étant détectés par un système de type TCSPC (Time Correlated Single Photon Counting).

Chaque photon est en fait détecté par rapport au départ de l'impulsion correspondante : sur la figure 1C, $t_i$ représente la durée écoulée entre chaque impulsion laser $I_i$ et l'instant de détection de chaque photon $p_i$.

Il est donc ensuite possible d'établir une répartition statistique ou histogramme du temps d'arrivée des photons, comme illustré sur la figure 1D, qui représente le nombre de photons de fluorescence détectés, en fonction du temps écoulé t par rapport à chaque impulsion laser. Cet histogramme représente une mesure expérimentale de la fonction $M_{sd}(t)$ précédemment évoquée, cette fonction $M_{sd}(t)$ étant l'histogramme théorique du temps d'arrivée des photons. On peut déterminer, à partir d'un tel histogramme, des paramètres statistiques, notamment l'intensité ou une moyenne du temps d'arrivée, ou temps moyen (il s'agit en fait de la moyenne des abscisses pondérée par les ordonnées de l'histogramme). Ainsi, pour chaque couple rassemblant une source et un détecteur, dit couple source détecteur et noté sd, on obtient un tel histogramme, que l'on assimile à la fonction $M_{sd}(t)$, suite à un grand nombre de mesures, ou acquisition.

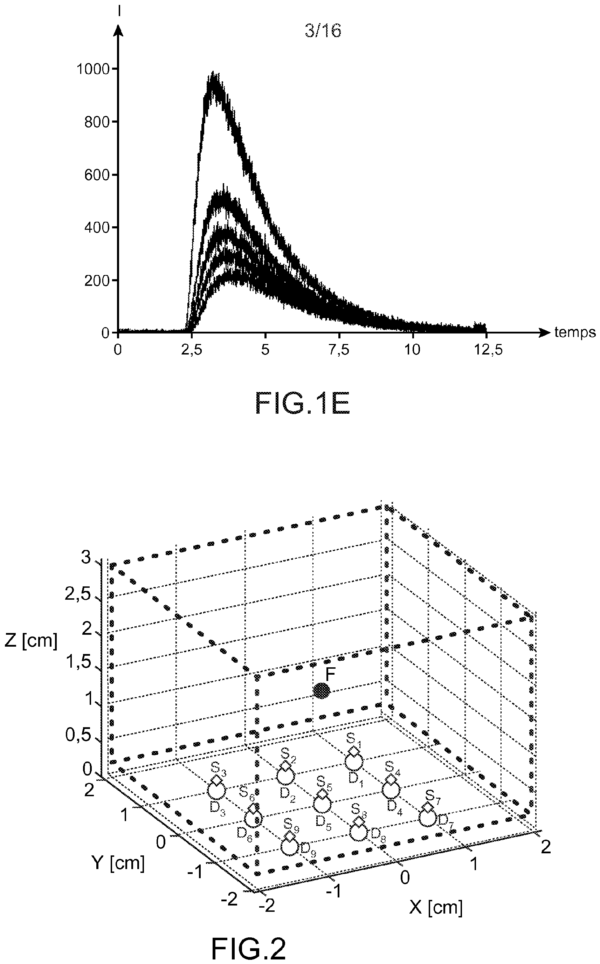

Une telle courbe $M_{sd}(t)$ qui, on le voit (voir également l'exemple de la figure 1E), permet d'utiliser toute l'information sur une large fenêtre temporelle, de part et d'autre du point d'intensité maximum (et pas seulement dans la partie montante du signal) peut donc ensuite être traitée pour en tirer des informations caractéristiques, tel que le temps d'arrivée, ou temps moyen comme on va l'expliquer ci-dessous.

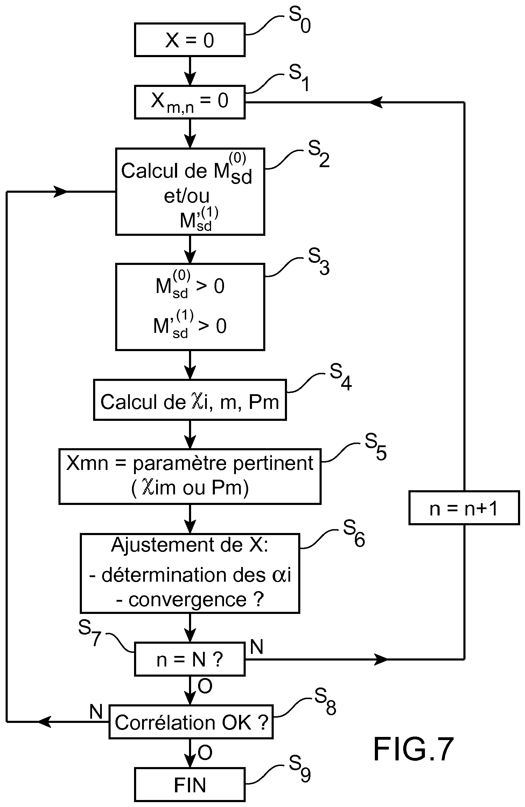

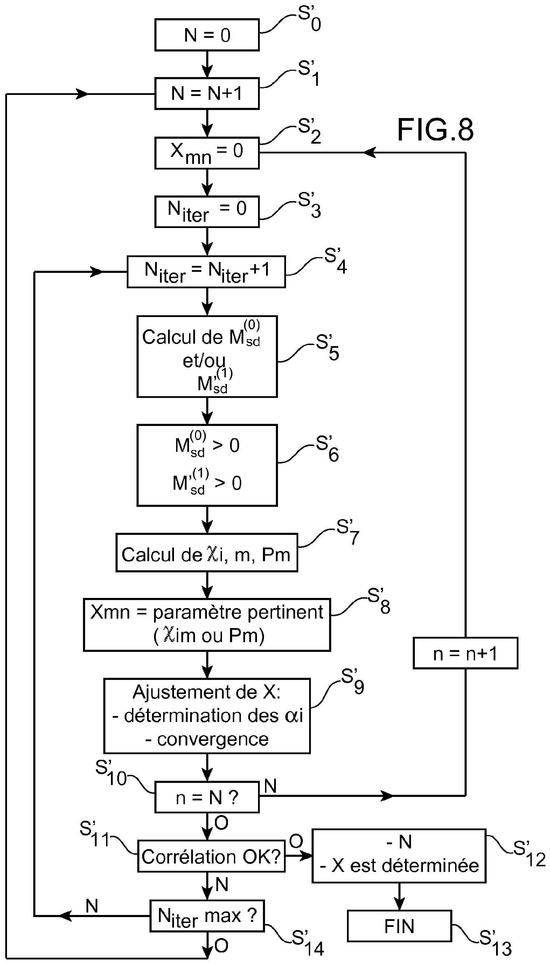

Des moyens électroniques 24 tels qu'un micro ordinateur ou un calculateur sont programmés pour mémoriser et traiter les données issues des détecteurs. Plus précisément une unité centrale 26 est programmée pour mettre en oeuvre un procédé de traitement selon l'invention : calculer un ou des paramètres $\chi^N_{i,m}$ ou $\chi_{3,m}$ ou une ou plusieurs fonctions de ces paramètres et/ou appliquer un procédé tel que par exemple décrit ci dessous en liaison avec l'une des figures 7 ou 8. Ces moyens 26 permettent éventuellement une ou des comparaisons de la ou des valeurs trouvées pour le ou les paramètres, avec une ou des valeurs seuils.

Un opérateur sélectionne un ou plusieurs de ces paramètres et/ou une ou plusieurs fonctions de ces paramètres, et le ou les calcule pour obtenir une première information sur une localisation d'au moins un fluorophore, éventuellement après avoir comparé les résultats du calcul à une ou des valeurs seuil ou à une ou des valeurs minimales ou à une ou des valeurs maximales.

Les moyens électroniques 24 permettent éventuellement un contrôle du déclenchement de la ou des sources de rayonnement et/ou du ou des détecteurs, par exemple une synchronisation de l'ensemble de ces moyens.

Un écran ou des moyens 27 d'affichage ou de visualisation permettent, après traitement, de représenter le positionnement du ou des fluorophores dans le milieu 2 examiné. Les moyens 24 et les moyens 27 d'affichage permettent également à un opérateur de déterminer un maillage tel qu'utilisé dans la présente demande, comme expliqué ci dessous.

Si le résultat d'une localisation ne convient ou n'est pas satisfaisant, par exemple par manque de précision, au moins un autre paramètre peut être sélectionné, ou au moins une itération peut être réalisée.

D'autres techniques de détection peuvent être employées, par exemple avec une caméra intensifiée, et par exemple une caméra intensifiée ultra-rapide de type « gated caméra » ; dans ce cas la caméra s'ouvre sur une porte temporelle, de largeur par exemple environ 200 ps, puis cette porte est décalée, par exemple de 25 ps en 25 ps.

La lumière d'excitation, à la longueur d'onde $\lambda_\chi$ excite les fluorophores $F_i$ qui réémet une lumière dite d'émission à la longueur d'onde $\lambda_m > \lambda_x$ avec une durée de vie $\tau$. Cette durée de vie correspond à la durée moyenne pendant laquelle les électrons excités restent dans cet état avant de retourner à leur état initial. Dans le cas ou l'on n'effectue pas d'imagerie de fluorescence, la méthode permet de localiser des absorbeurs dans le milieu, c'est-à-dire des lieux dans lesquels le coefficient d'absorption du milieu est plus élevé que le coefficient d'absorption moyen du milieu. Dans la suite de la demande, on pourra assimiler un tel absorbeur à un fluorophore, en considérant que sa longueur d'onde de fluorescence, ou longueur d'onde d'émission, égale à la longueur d'onde d'excitation, et en considérant également que la durée de fluorescence est nulle, soit $\tau = 0$. Dans un tel cas, le filtre éventuellement placé en amont du détecteur sera adapté à la longueur d'onde d'excitation.

Quels que soient le milieu considéré et la géométrie, une ou des inclusions fluorescentes sont situées dans celui-ci, qui présente une vitesse de propagation de la lumière c ($=3*10^8$m/s/n, n = indice optique), un coefficient de diffusion D et un coefficient d'absorption $\mu_a$.

Le milieu environnant 2 le fluorophore $F_1$ peut avoir toute forme de géométrie : par exemple, il est de type infini.

Il peut aussi être de type semi infini, limité par une paroi. Dans un tel cas, les fibres optiques d'excitation et d'émission peuvent être placées à l'interface du milieu diffusant, ou à faible profondeur, de l'ordre de quelques mm.

Un milieu semi infini, pour lequel chacune des extrémités de fibres d'amenée du signal d'excitation et de prélèvement du signal de fluorescence est disposée à plus de 1 cm, ou de 1,5 cm ou de 2 cm de la paroi qui limite ce milieu peut être traité, du point de vue du procédé selon l'invention, comme un milieu infini.

Le milieu environnant peut encore former une tranche (géométrie dite « slab »), limitée par deux surface parallèles.

L'invention s'applique aussi à un milieu de forme quelconque, dont la surface extérieure est décomposée en une série de facettes planes.

Dans tous les cas elle est particulièrement bien adaptée au cas où la géométrie est en réflexion, dans lequel la ou les sources et le ou les détecteurs se trouvent sur la même face de l'objet ou du milieu étudié).

Les fonctions de Green $G_s$(r,t) d'un dispositif selon l'invention peuvent être déterminées en utilisant une simulation Monte-Carlo, par exemple pour résoudre l'équation de transfert radiatif ou, le plus souvent, en résolvant l'équation de diffusion (1) auquel on ajoute des conditions de bords (condition de courant partiel (Robin) ou condition de bord extrapolé (Dirichlet)), comme expliqué dans l'article de S.R. ARRIDGE :"Optical tomography in medical imaging", Inverse Problems, april 1999, vol 15(2), R41-R93. Ces déterminations ou simulations peuvent être réalisées à l'aide de moyens informatiques, tels que les moyens 26, 27 des figures 1A et 1B. Les moments des fonctions de Green Gs(r,t), tels que précédemment définis (1'), peuvent être obtenus en résolvant itérativement l'équation (2).

Selon un mode de réalisation non revendiqué , un fluorophore est un fluorophore unique $F_1$, ou une pluralité de fluorophores groupés sensiblement en un même lieu.

Selon un autre mode de réalisation, le fluorophore est constitué de N fluorophores répartis dans des lieux différents.

A l'aide de mesures optiques résolues en temps, on cherche à localiser ces inclusions fluorescentes, c'est-à-dire à reconstruire la carte de fluorescence.

Selon l'invention, on reconstruit la carte de fluorescence par une méthode efficace d'un point de vue informatique, et qui a la propriété de concentrer la fluorescence sur de petits supports. Le terme "petits supports" représente le fait que le ou les fluorophores sont répartis de façon discrète dans certains lieux du milieu étudié. En effet, les inventeurs ont considéré que ce type d'a priori s'appliquait bien à la reconstruction de fluorophores dans des tissus diffusants, notamment dans certaines applications liées à la détection précoce de tumeurs cancéreuses dans des organes, tels la prostate, le sein, les testicules, le cerveau...

Dans un 1$^{er}$ mode de réalisation non revendiqué, mais dont des aspects peuvent être combinés avec l'invention, on considère le cas d'un fluorophore unique.

Selon le procédé utilisé, on met en oeuvre les informations sur $M^0_{sd}$ et $M^1_{sd}$ (déjà définis ci-dessus, voir par exemple équations 11-14) afin de reconstruire la position des fluorophores, lesquels émettent le rayonnement de fluorescence.

Dans tous les cas, on maille le volume de recherche du fluorophore en M mailles (ou voxels) m, dont la taille dépend de la précision souhaitée : plus la précision est élevée, plus le volume individuel d'une maille est faible et plus le nombre de mailles est élevé.

Ce maillage peut être décidé par un opérateur du système informatique 24.

Selon l'invention, on utilise un ou plusieurs premiers $\chi_{im}$ paramètres dits paramètres de base(ou critères, mais on utilisera par la suite de l'expression « paramètres ») qui sont définis ci-dessous, ou une combinaison de ceux-ci, cette combinaison pouvant être alors appelé paramètre combiné Pm. Selon ce mode de réalisation, l'indice m désigne un voxel.

Chacun de ces paramètres de base dépend de la variable m, donc de la maille sélectionnée dans le volume. On sélectionne une à une toutes les mailles, et pour chaque maille, on calcule le paramètre, qui est une somme de contributions de tous les couples (source, détecteur) possibles du système ou d'une partie de l'ensemble de ces couples. Autrement dit, chaque paramètre représente la somme, pour différentes acquisitions, chaque acquisition correpondant à un couple source-détecteur, d'une combinaison entre une grandeur (ici et dans la suite du texte, cette expression est équivalente à « fonction » ou « quantité » ou « paramètre ») établie à partir d'au moins un moment de $M_{sd}(t)$, et l'estimation de cette grandeur, cette estimation étant réalisée en considérant un fluorophore unique positionné dans le voxel m.

Par combinaison, on entend toute opération arithmétique, linéaire ou non. On verra dans la suite de la description que cette combinaison peut prendre par exemple la forme d'une soustraction ou d'un produit.

On verra également que la grandeur établie à partir d'au moins un moment de la fonction $M_{sd}(t)$ peut-être le moment d'ordre 0 de cette fonction, ou encore le moment normé d'ordre 1, auquel on soustrait des grandeurs (ou fonctions) temporelles connues et supposées constantes.

Selon un mode préféré, cette grandeur peut-être le moment d'ordre 0 de $M_{sd}(t)$, noté $M^{(0)}_{sd}$.

Ainsi on définit un premier paramètre de base , $\chi_{1m}$:

$$\chi_{1m} = \min_{\alpha_m} \sum_{sd} \frac{\left(M_{sd}{}^0 - \alpha_m . G_{sm} G_{md}\right)^2}{\sigma^2\left(M_{sd}{}^0\right)}$$

$\chi_{1m}$, peut être considéré comme un résidu entre la mesure du moment $M^{(0)}_{sd}$, et son estimation, donnée par le produit $\alpha_m G_{sm} G_{md}$. $\chi_{1m}$ est minimal pour les mailles m donnant le meilleur positionnement du fluorophore. Sa valeur résulte du calcul de la valeur minimum de la somme indiquée ci-dessus, pour un ensemble de coefficients $\alpha_m$. Autrement dit, on recherche l'ensemble des coefficients $\alpha_m$ qui minimise la somme ci-dessus.

Le terme $\sigma^2(M_{sd}{}^0)$ figurant au dénominateur est optionnel. Il s'agit d'une estimation de l'incertitude associée à la détermination de $M_{sdi}{}^0$. Si $M_{sd}{}^0$ est le nombre de photons détecté par le couple de détecteurs (s, d), et si le bruit est Poissonien, alors $\sigma^2(M_{sd}{}^0) = M_{sd}{}^0$

Ainsi, chaque élément $(M_{sd}{}^0 - \alpha_m G_{sm} G_{md})^2$ de la somme représente la différence entre :

- la valeur de l'intensité mesurée $M_{sd}{}^0$ pour le couple (source, détecteur) sélectionné, lorsque la source émet une impulsion et le détecteur détecte un signal de fluorescence émis par le fluorophore,
- et une estimation du moment $M_{sd}{}^0$ d'ordre zéro, obtenue à l'aide des fonctions de Green $G_{sm}$ et $G_{md}$, qui peuvent être estimées de la manière déjà décrite ci-dessus, par exemple à l'aide des moyens 24.

Toujours selon un mode préféré de réalisation, la grandeur établie à partir d'au moins un moment de $M_{sd}(t)$ est le moment normé d'ordre 1, noté $T_{sd}$, auquel on retranche le temps de la fonction la source Ts, le temps de réponse du détecteur Td, ainsi que le temps de fluorescence $\tau$. On utilise dans ce cas un deuxième paramètre de base, $\chi_{2m}$ :

$$\chi_{2m} = \sum_{s,d} \frac{\left(\left(T_{sd} - T_s - \tau - T_d\right) - \left(T_{sm} + T_{dm}\right)\right)^2}{\sigma^2(T_{sd})}$$

$\chi_{2m}$, appelé aussi critère de résidu des mesures de temps de vol, est lui aussi petit pour des mailles vérifiant un bon accord temporel, et donc contribuant à donner un meilleur positionnement du fluorophore. Plus spécifiquement, $\chi_{2m}$ correspond à une différence (ou un résidu) entre, pour au moins une acquisition, le temps de vol moyen source détecteur mesuré (moment normé d'ordre 1 de M(t)) et ce même moment d'ordre 1 normé modélisé, le fluorophore étant alors supposé correspondre au voxel m.

Dans l'expression ci-dessus de ce paramètre $\chi_2 m$:

- $T_{sd}$ est le temps de vol source-détecteur mesuré, il s'agit du moment normé d'ordre 1 de le mesure.
- $T_s$ est le temps moyen de la fonction source, précédemment évoqué;
- $T_d$ est le temps de réponse du détecteur, précédemment évoqué;
- $\tau$ est la durée de vie du fluorophore;
- $T_{sm}$ est la durée moyenne du trajet source-fluorophore, (ou source - voxel (m)) ;

- $T_{dm}$ est la durée moyenne du trajet fluorophore - détecteur (ou détecteur - voxel (m)).

Le terme (Tsd - Ts - Td - $\tau$) représente le temps de vol "net", c'est-à-dire le temps de vol mesuré, auquel on soustrait les temps moyen de la fonction source, de la réponse du détecteur et la durée de vie de fluorescence. Ce temps de vol net représente la durée du trajet de la lumière entre la source, le fluorophore et le détecteur.

Tsm + Tdm représente une estimation de ce temps de vol net lorsque le fluorophore est présent dans le voxel m.

Les grandeurs $T_s$, $T_d$, $\tau$ sont des données en principe connues : $\tau$ dépend du fluorophore, tandis que la somme Ts + $T_d$ peut être obtenue par un essai de calibration, comme précédemment décrit. $T_{sm}$ et $T_{dm}$ sont modélisées, soit analytiquement, soit en résolvant l'équation (2), en considérant que le fluorophore est unique et situé dans le voxel m.

De façon préférentielle, et comme c'est le cas dans la formule ci-dessus, le paramètre $\chi_{2m}$ peut être normé par une grandeur (ici et dans la suite du texte, cette expression est équivalente à « fonction » ou « paramètre ») statistique relative à la distribution de $T_{sd}$. Cette grandeur statistique joue le rôle d'indicateur de confiance. On a choisi ici $\sigma^2 (T_{sd})$, mais une autre grandeur statistique (par exemple écart type ou variance) pourrait être choisie, pour indiquer la confiance qu'on attribue à une distribution, mais la normalisation par la variance est optimale. Cette grandeur statistique permet de pondérer les mesures correspondant à un couple source-détecteur par rapport à d'autres mesures correspondant à un autre couple source-détecteur : plus la distribution de $T_{sd}$ a un écart-type faible, plus les mesures issues de ce couple source-détecteur peuvent être considérées comme fiables et plus on pourra leur donner de l'importance dans la détermination du second coefficient, qui est expliquée plus loin. La grandeur $\sigma^2 (T_{sd})$ peut-être estimée aisément à partir d'une largeur $\Delta T_{sd}$ de la fonction $M_{sd}(t)$ selon les équations suivantes :

$$\Delta T_{sd} = \sqrt{\frac{\sum_i (t_i - T_{sd})^2 M_{sd}(t_i)}{M_{sd}^{(0)}}}$$

$$\sigma^2(T_{sd}) = \frac{\Delta T_{sd}^{\;2}}{M_{sd}^{(0)}}$$

où :

- ti désigne l'abscisse i de la fonction $M_{sd}$ (t)

Comme pour le premier paramètre de base $\chi_{1m}$, $\chi_{2m}$, est minimal pour les mailles m dans lesquelles le fluorophore est effectivement positionné.

Ainsi, lorsqu'on utilise ces deux premiers paramètres $\chi_{1m}$ et $\chi_{2m}$, les mailles du volume, pour lesquelles ils sont tous deux minimum sont des mailles dans lesquelles le fluorophore peut très vraisemblablement se situer.

De plus, les inventeurs ont mis en évidence l'intérêt particulier à utiliser conjointement ces deux paramètres de base $\chi_{1m}$ et $\chi_{2m}$. En effet, lorsque les sources et les détecteurs sont situés dans un plan P (ce qui est typiquement le cas pour une configuration en réflexion), le premier paramètre de base $\chi_{1m}$ donne une information particulièrement pertinente quant aux coordonnées du fluorophore dans ce plan. Autrement dit, les voxels m pour lesquels le coefficient $\chi_{1m}$ est minimum sont généralement distribués selon la direction perpendiculaire à ce plan, mais leurs coordonnées dans ce plan sont peu dispersées.

De façon complémentaire, et sans qu'on puisse s'y attendre, le deuxième paramètre de base $\chi_{2m}$ donne une information particulièrement pertinente sur la position du fluorophore dans la direction perpendiculaire à ce plan. Ainsi, un paramètre $\chi_{2m}$, combinant une mesure et une estimation du temps de vol, donne une indication relative à la profondeur du fluorophore par rapport à un plan formé par les sources et détecteurs. Autrement dit, les voxels m pour lesquels le coefficient $\chi_{2m}$ est minimum sont généralement distribués de façon sensiblement parallèle au plan formé par les sources et les détecteurs, et leurs coordonnées selon la direction perpendiculaire à ce plan sont peu dispersées. Ces voxels sont alors situés à une même profondeur par rapport à l'endroit ou sont situés les sources et détecteurs.

On comprend alors l'intérêt de combiner ces deux premiers paramètres de base : la probabilité de présence du fluorophore dans un voxel m sera d'autant plus élevée que, pour ce voxel, le premier paramètre de base $\chi_{1m}$ et le deuxième paramètre de base $\chi_{2m}$ sont minimum. Du fait de la complémentarité existant entre ces deux premiers paramètres de base $\chi_{1m}$ et $\chi_{2m}$, on obtiendra une information pertinente quant aux coordonnées du fluorophore dans le plan défini par les sources et les détecteurs (par le premier paramètre de base $\chi_{1m}$), mais également une information pertinente quant aux coordonnées du fluorophore dans la direction orthogonale à ce plan, c'est-à-dire à la profondeur (par le deuxième paramètre de base $\chi_{2m}$). Les coordonnées du voxel susceptible de contenir le fluorophore seront ainsi définies.

Un troisième paramètre de base $\chi_{3m}$ peut être constitué en considérant que la grandeur établie à partie de la distribution Msd(t) est le moment d'ordre 0, de même que lors de la détermination du premier paramètre de base. On effectue ensuite une combinaison de cette grandeur avec l'estimation de cette grandeur, ladite estimation étant réalisée en considérant que le fluorophore est unique et localisé dans le voxel m. Mais cette combinaison n'est ici pas une différence, mais une corrélation, autrement dit un produit. Ainsi, ce troisième paramètre de base correspond à la corrélation, pour au moins un couple source-détecteur, entre une valeur de l'intensité mesurée ($I_{sd}$ = $M^{(0)}$(t)) déterminée par le moment d'ordre 0 de M(t), et une valeur de ce moment d'ordre 0 estimé (le produit $G_{sm}.G_{dm}$). Ce coefficient de corrélation correspond à la somme, pour chaque couple source-détecteur modélisé, du produit entre la valeur de $I_{sd}$ mesurée (intensité pour une excitation par la source S et une détection par le détecteur D) et l'intensité modélisée :

$$\chi_{3m} = \frac{\sum\limits_{sd} I_{sd} G_{sm} G_{dm}}{\sqrt{\sum\limits_{s,d} G_{sm}^{2}.G_{dm}^{2}}},$$

m étant l'indice correspondant au voxel considéré, s et d représentent la source et le détecteur considérés.

On peut également normer ce coefficient:

$$\frac{\sum\limits_{sd} I_{sd} G_{sm} G_{dm}}{\sqrt{\sum\limits_{s,d} G_{sm}^{2}.G_{dm}^{2}}}$$

en utilisant :

$$\chi_{3m} = \frac{\sum\limits_{s,d} I_{sd}.G_{sm}.G_{dm}}{\sqrt{\sum\limits_{s,d} I_{sd}^{2}}.\sqrt{\sum\limits_{s,d} G_{sm}^{2}.G_{dm}^{2}}}$$

ce coefficient normé étant alors compris entre 0 et 1.

A la différence des deux paramètres précédents, $\chi_{3m}$, est maximal pour les mailles m donnant le meilleur positionnement du fluorophore, notamment dans un plan sensiblement parallèle à celui formé par les sources et les détecteurs.

Pour mettre en oeuvre un procédé selon l'invention, on sélectionne au moins l'un des paramètres de base, ci-dessus, et l'on constitue une combinaison $P_m$ de ces paramètres. Ainsi, Pm peut être appelé paramètre combiné, résultant d'une combinaison d'au moins un premier paramètre (pour un seul paramètre, on parlerait plus volontiers d'une « fonction » de ce paramètre, mais on utilise dans la présente demande, par souci de simplification, l'expression générale « combinaison »). Une telle combinaison Pm est elle aussi fonction de m, c'est-à-dire du voxel considéré.

Une telle combinaison $P_m$ de ces paramètres peut être, selon un mode préféré, la somme du premier et du deuxième paramètres de base :

$$\chi_{1m} + \chi_{2m}$$

Ainsi, lorsqu'on utilise ce paramètre combiné $P_m = \chi_{1m} + \chi_{2m}$, les mailles du volume, pour lesquelles $P_m$ est minimum, sont des mailles dans lesquelles le fluorophore peut très vraisemblablement se situer.

Un autre exemple d'une combinaison des paramètres de base, peut être par exemple le produit du premier et du deuxième paramètre :

$$\chi_{1m} \cdot \chi_{2m}$$

Là encore, ce paramètre combiné $P_m$ est minimum pour la/les maille(s) dans la/lesquelles le fluorophore est très vraisemblablement situé.

Encore un autre exemple d'une combinaison des paramètres de base, peut être par exemple le rapport du troisième et du deuxième paramètres de base :

$$\chi_{3m}/\chi_{2m}$$

Ce paramètre combiné $P_m$ est maximum pour la/les maille(s) dans la/lesquelles le fluorophore est très vraisemblablement situé, puisque le troisième paramètre est alors minimum, tandis que le deuxième est maximum. Par contre, on cherchera à maximiser le paramètre combiné inverse, $\chi_{2m}/\chi_{3m}$, si c'est celui-ci qui est retenu en tant que paramètre combiné.

On peut également ne retenir qu'un seul paramètre, fonction de l'un des paramètres de base ci-dessus, par exemple le carré de l'un de ces paramètres, ou son inverse.

Pour former un paramètre combiné, on peut en outre utiliser au moins deux des paramètres de base, l'un au moins d'entre eux étant l'argument d'une fonction non linéaire. Par exemple, on peut utiliser le produit $\chi_{1m}^2 \cdot \chi_{2m}$, le produit du carrée du premier par le deuxième. Un autre exemple est le rapport $\chi_{2m}^2/\chi_{3m}$.

On peut également combiner les trois paramètres $\chi_{1m}$, $\chi_{2m}$ et $\chi_{3m}$ ci-dessus, en prenant en compte le fait que les deux premiers voient leur valeur croître, pour un certain voxel, avec la probabilité de présence du fluorophore dans ce voxel, tandis que le dernier voit sa valeur décroître.

Dans ce cas d'un seul fluorophore, chaque valeur du paramètre de base ou du paramètre combiné retenu, pour un voxel donné, peut-être rangée dans un vecteur X (M,1), chaque coordonnée du vecteur représentant la valeur $P_m$ du voxel m ($1 < i < m$). Un tel vecteur peut-être appelé carte de fluorescence.

Quel que soit le paramètre retenu (qu'il s'agisse d'un premier paramètre de base $\chi_{im}$ (i désignant l'indice du paramètre de base) ou qu'il s'agisse d'un paramètre combiné Pm, on cherchera à déterminer si celui-ci a une valeur supérieure ou inférieure à une valeur donnée $P_{limite}$, afin de déterminer si le voxel m correspondant est celui dans lequel le fluorophore est probablement, ou même très probablement, situé, ou pas. $P_{limite}$ est par exemple la moitié de la valeur maximum prise par ce paramètre sur l'ensemble des voxels considérées, et, pour le cas d'un fluorophore unique, on pourra par exemple retenir le voxel pour lequel le paramètre a la valeur maximale.

En variante, $P_{limite}$ est égal au minimum à la moitié de la valeur maximum prise par ce paramètre sur l'ensemble des voxels considérées et, pour le cas d'un fluorophore unique, on pourra par exemple retenir le voxel pour lequel le paramètre a la valeur minimale.

En fonction de la valeur que le paramètre retenu prendre pour un voxel $m_0$ donné, ce dernier peut apparaître sur une image représentative du milieu considéré, par exemple sur l'écran 27, avec une certaine nuance de couleur en fonction de la valeur de $P_{m0}$. En variante, ce sont les seuls voxels pour lesquels le paramètre retenu est supérieur ou inférieur à la valeur de seuil sélectionnée qui seront représentés sur cette image.

Selon un exemple de réalisation, on utilise le paramètre $\chi_{3m}/\chi_{2m}$, égal au rapport du troisième au deuxième.

On prend l'exemple de la configuration de la figure 2 qui est un schéma d'une configuration utilisée pour des tests; cette configuration comporte 3x3 sources $S_1$ - $S_9$, positionnées dans le plan z = 0, et 3x3 détecteurs $D_1$ - Dg, positionnées dans le même plan que les sources. Le fluorophore F est positionné dans le plan z=1.6cm.

On réalise un éclairement avec les 9 sources et il y a formation d'un signal de fluorescence par les 9 détecteurs.

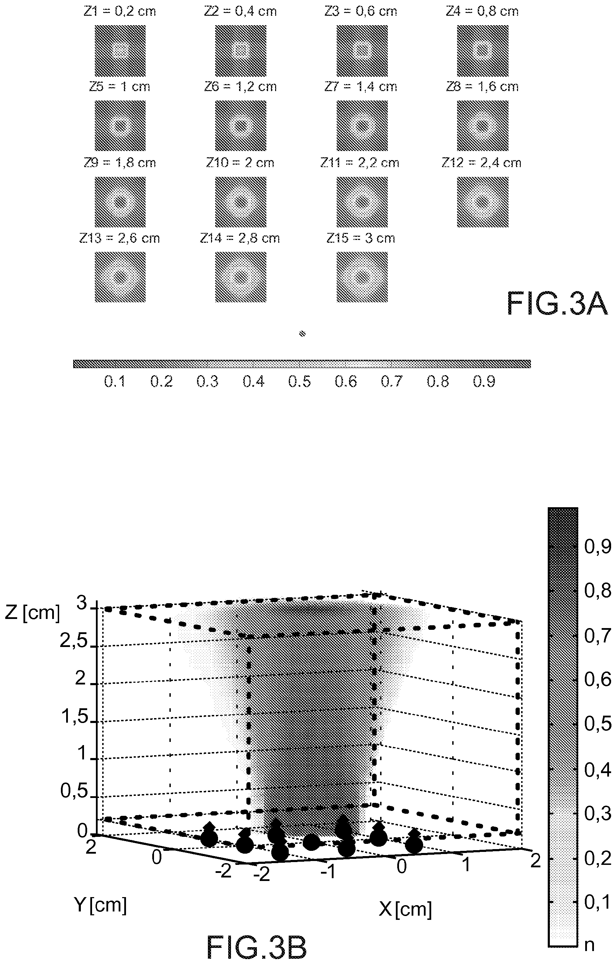

On applique la méthode présentée ci-dessus à cette configuration de la figure 2, seul le paramètre de base $\chi_{3m}$ étant sélectionné, on obtient la carte des corrélations des figures 3A et 3B.

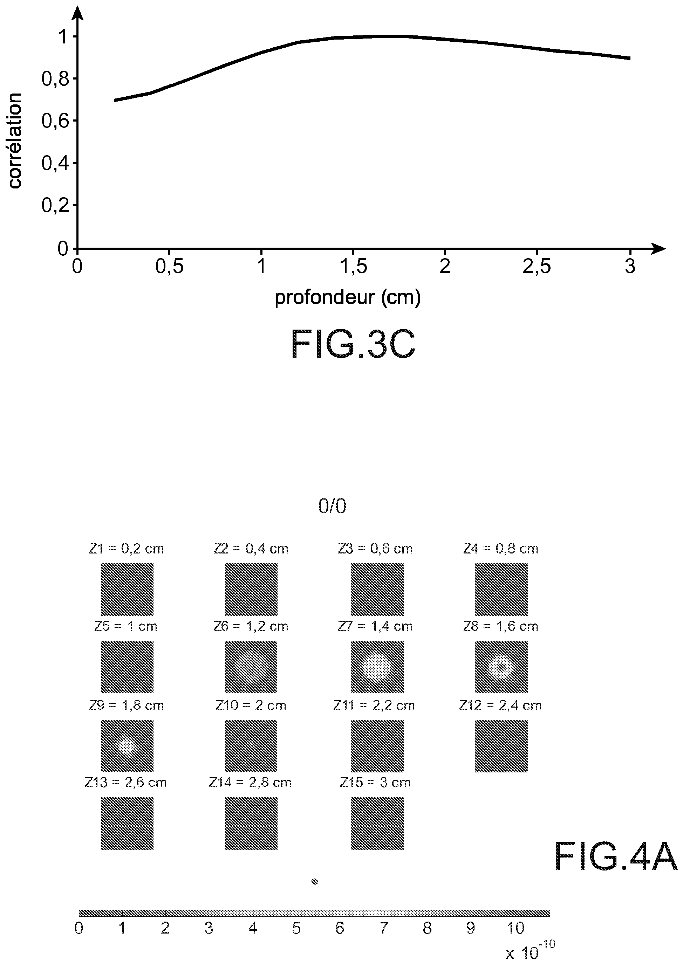

On voit, notamment en figure 3B, que la position est relativement bien déterminée suivant les axes x et y. Toutefois, la position suivant z n'est pas précise du tout. En figure 3A sont représentées des coupes de la figure 3B à différentes altitudes de z : la zone de localisation identifiée pour le fluorophore y apparaît comme plus claire : cette zone s'étend le long de la direction z, c'est pourquoi on voit sa trace à différentes altitudes z sur la figure 3A (c'est le même principe sur les figures 4A, 5A, 5B, 11C et 12C décrites ci dessous).

En figure 3C est représentée la valeur de $\chi_{3m}$ en fonction de la profondeur, le long d'une droite verticale (parallèle à z) et passant par le fluorophore. La forme de la courbe résultante, très plate, confirme cette imprécision : le paramètre $\chi_{3m}$ a presque la même valeur partout, cette valeur est proche de 1, donc le fluorophore peut être partout.

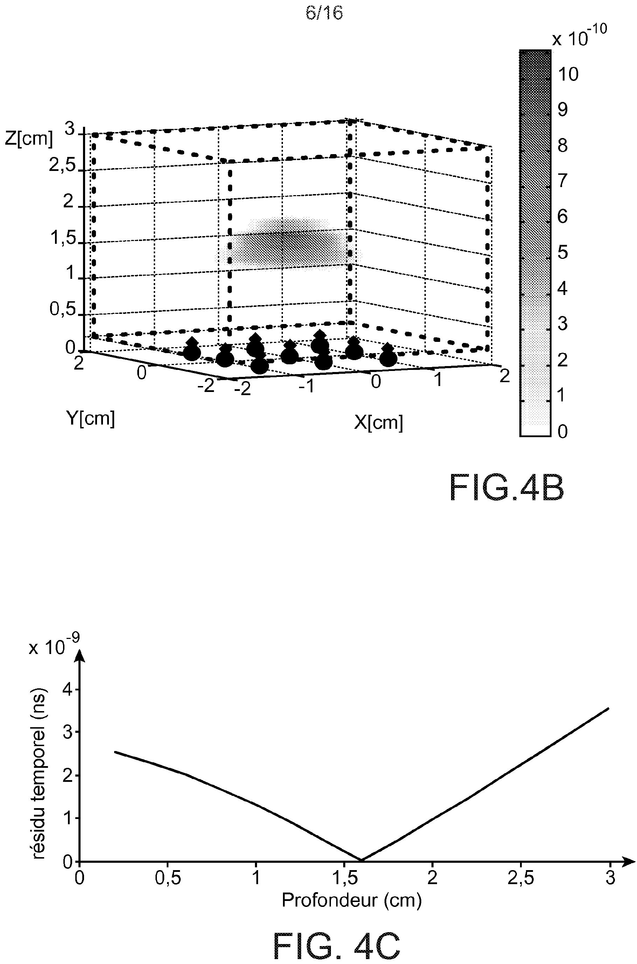

La carte du critère $-\chi_{2m}$, avec un facteur $\sigma_m$ pris constant et égal à 1 pour le besoin de la simulation, est représentée en figures 4A et 4B. Plus exactement, c'est le critère max $(-\chi_{2m})/6 - \chi_{2m}$ qui est en fait représenté, limité aux valeurs positives. En figure 4A sont représentées des coupes de la figure 4B à différentes altitudes de z.

On voit en figure 4B qu'on obtient cette fois un résultat plus précis suivant z que suivant x et y.

En figure 4C est représentée la valeur du paramètre combiné $P_{m1}$ = max $(-\chi_{2m})/6 - \chi_{2m}$ en fonction de la profondeur, le long d'une droite verticale (parallèle à z) et passant par le fluorophore. La forme de la courbe résultante, minimale au voisinage d'une valeur particulière (ici proche de z = 1,5 cm), montre qu'une localisation précise suivant z peut être obtenue en utilisant ce paramètre combiné.

On peut en outre utiliser un paramètre $p_m$ qui combine les deux paramètres ci-dessus. Par exemple :

$$P_{m2} = \chi_{3m} / \chi_{2m},$$

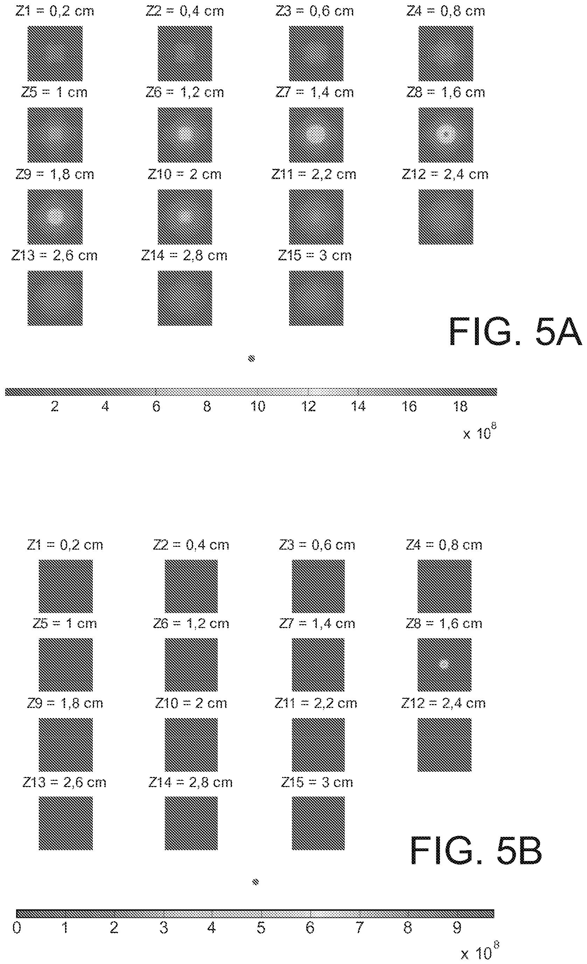

On obtient alors un résultat précis à la fois dans le plan x, y mais aussi pour z, comme illustré en figures 5A et 5B, qui sont là encore des coupes successives suivant différentes altitudes z.

On voit là qu'on a donc pu positionner correctement le fluorophore.

Bien que l'on ait décrit ici la détermination des paramètres $\chi_{1m}$, $\chi_{2m}$, et $\chi_{3m}$, à partir du moment d'ordre 0 ou à partir du moment normé d'ordre 1 d'au moins une distribution $M_{sd}(t)$, on comprend que d'autres paramètres de base pourraient être déterminés, combinant d'une part une grandeur établie à partir d'au moins un moment de $M_{sd}(t)$, et, d'autre part, une estimation de cette grandeur, cette estimation étant réalisée en considérant que le fluorophore est unique et situé dans le voxel m.

Dans un autre mode de réalisation non revendiqué, mais dont des aspects peuvent être combinés avec l'invention, on traite le cas de N (>1) fluorophores, N étant un nombre connu.

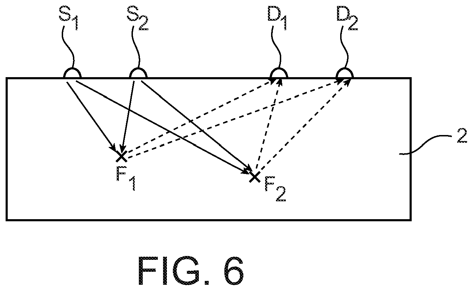

On illustre ce cas en figure 6, dans lequel un milieu 2 contient 2 fluorophores $F_1$, $F_2$ et est muni de deux sources $S_1$, $S_2$ et de 2 détecteurs $D_1$, $D_2$ pour détecter la fluorescence émise par les fluorophores.

Lorsqu'un éclairement est réalisé par la source $S_1$, il se produit une excitation des 2 fluorophores, qui réémettent chacun un rayonnement de fluorescence capté par les détecteurs $D_1$, $D_2$.

Il en va de même lorsqu'un éclairement est réalisé par la source $S_2$ : le signal de chacun des deux détecteurs contient une contribution de chacun des deux fluorophores.

Il s'agit donc de pouvoir identifier, à l'aide des signaux produits par les deux détecteurs $D_1$, $D_2$, les positions des deux fluorophores $F_1$, $F_2$.

On part donc des intensités et des temps de vol déterminés à partir des signaux fournis par les deux détecteurs.

On procède ensuite, pour chaque fluorophore, au calcul ou à l'estimation d'un ou plusieurs paramètres, de base ou combiné, ayant la même forme que ceux qui ont déjà été décrits ci-dessus. Mais on tient compte des valeur de ces mêmes paramètres qui ont déjà été calculées pour un ou plusieurs autres fluorophores.

Autrement dit, dans les formules qui donnent les valeurs des paramètres de base, on va remplacer les valeurs de moments $M^{(0)}_{sd}$ et $M^{(1)}_{sd}$ pour chaque couple (source, détecteur) par une valeur corrigée $M'^{(0)}_{sd}$ et $M'^{(1)}_{sd}$ qui tient compte des contributions des fluorophores déjà identifiés. Les valeurs corrigées sont égales à la différence entre les moments mesurés et la contribution estimée, à ces moments, des fluorophores déjà identifiés ou localisés. On procède ainsi par itération.

Dans cette variante du procédé, appliqué au cas de N fluorophores, on reconstruit une matrice X(M,N), N étant le nombre de fluorophores, M étant le nombre de voxels. Chaque élément de la matrice $X_{mn}$ représente le coefficient combiné $P_m$ correspondant au voxel m ($1<m<M$) et au fluorophore n ($1<n\leq N$). Cette matrice est une association de N vecteurs colonnes X(M,1), c'est-à-dire de N cartes de fluorescence.

Comme précédemment, les mesures $M_{sd}(t)$ correspondant à différents couples sources-détecteurs sont réalisées préalablement, ce qui permet de déterminer les moments $M^{(0)}_{sd}$ et $M^{(1)}_{sd}$ pour chaque couple source détecteur.

Le procédé est maintenant expliqué en liaison avec la figure 7.

Au départ (S0), tous les éléments de la matrice sont initialisés à 0. On connaît N (nombre de fluorophores présents à priori dans le milieu diffusant), et M (nombre de voxels discrétisant le milieu).

Pour chaque valeur de n :

- on met d'abord les coefficients ($1<m<M$) de la colonne n à 0 (étape S1) ;
- on calcule (étape S2) le reliquat des mesures, à partir des formules 10 et 12 ci-dessus, discrétisées sur un maillage et dont les mailles sont indexées par m) :

$$M'^{(0)}_{sd} = M^{(0)}_{sd} - S^{(0)} D^{(0)} \sum_m \sum_n .G_{sm}.X_{mn}.G_{md}.$$

$$M'^{(1)}_{sd} = M^{(1)}_{sd} - S^{(0)} D^{(0)} \sum_m \sum_n .G_{sm}.X_{mn}.G_{md}.(T_s + T_{sm} + \tau + T_{md} + T_d)$$

Dans ces formules $G_{sm}$ et $G_{md}$ sont les fonctions de Green simulées (dont il a déjà été question précédemment)

et $X_{mn}$ est l'élément (m,n) de la matrice X, précédemment initialisé à 0, mais comportant des termes déjà estimés dès lors que n>1; on force, $M'^{(0)}_{sd}$ et $M'^{(1)}_{sd}$ à être strictement positifs, par mise à 0 des valeurs strictement négatives (étape S3).$S^{(0)}$ et $D^{(0)}$ ont déjà été définis.

Chaque valeur de $M'^{(0)}_{sd}$ est l'écart entre la valeur mesurée de $M^{(0)}_{sd}$ et la valeur estimée à l'aide des valeurs $G_{sm}$ et $G_{md}$, appliquées au coefficient courant $X_{mn}$, tel qu'il résulte de l'itération précédente (éventuellement de la mise à zéro initiale).

On peut également dire :

- que $M'^{(0)}_{sd}$ est l'intensité mesurée $M_{sd}^0$ pour chaque couple (source, détecteur) sélectionné, corrigée de l'estimation de la contribution à l'intensité des fluorophores déjà localisés, pour chacun desquels au moins un coefficient de la colonne correspondante de la matrice X est non nul;
- tandis que $M'^{(1)}_{sd}$ est le moment d'ordre 1 moyen source détecteur mesuré (moment normé d'ordre 1 de M(t)), corrigée de l'estimation de la contribution au temps de vol des fluorophores déjà localisés, là encore, pour chacun desquels au moins un coefficient de la colonne correspondante de la matrice X est non nul.

On peut ensuite (étape S4) déterminer, pour chaque voxel m et à l'aide de $M'^{(0)}_{sd}$ et $M'^{(1)}_{sd}$, au moins un des paramètres de base $\chi_{im}$ (par exemple $\chi_{1m}$, $\chi_{2m}$, $\chi_{3m}$) et éventuellement au moins un paramètre combiné $P_m$, $P_m$ ayant la forme déjà été définie précédemment pour le cas d'un seul fluorophore.

Ainsi, $\chi_{1m}$ est déterminé en remplaçant $M^{(0)}_{sd}$ par $M'^{(0)}_{sd}$ et $\chi_{2m}$ est déterminé en remplaçant $T_{sd}$ par $M'^{(1)}_{sd}/M'^{(0)}_{sd}$, tandis que $\chi_{3m}$ est déterminé en remplaçant $I_{sd}$ par $M'^{(0)}_{sd}$

Ainsi, n étant fixé, pour tout m (étape S5) :

$X(m, n) = P_m$ ou $\chi_{im}$ ou $\chi'_{im}$ (i = 1 - 3).

On peut éventuellement procéder à un ajustement des termes de la matrice X (étape S6) en attribuant un coefficient multiplicatif $\alpha_i$ à chacune des N colonnes de la matrice X en cours de constitution en tentant de faire converger la somme suivante vers $M^{(0)}_{sd}$:

$$\sum_m \sum_n G_{sm} X_{mn} G_{md} \alpha_n \approx M^{(0)}_{sd}$$

La convergence peut être obtenue par une méthode de moindre carrés. Cette étape permet d'améliorer significativement la qualité des résultats.

La matrice X est ajustée en multipliant chaque terme d'une colonne n par le coefficient $\alpha_n$. Précisons qu'on peut assimiler ces coefficients $\alpha_n$ à des intensités du signal de fluorescence.

On incrémente ensuite n (étape S7) et on retourne à l'étape S1 (où tous les éléments de la colonne n sont initialisés à 0). Si il n'y a pas d'étape S6 de test de convergence, on passe de S5 à S7.

Lorsque la matrice X a été constituée (n = N, étape S7) par la boucle précédente, on peut vérifier (étape S8) que $\sum_m \sum_n G_{sm} X_{mn} G_{md} \alpha_n$ est proche de $M^{(0)}_{sd}$.

Si la corrélation n'est pas satisfaisante, on réitère la boucle ci-dessus, à partir de l'étape S2 en prenant X, qui vient d'être calculée, pour matrice initiale. La corrélation peut comporter par exemple un test dit d'écart résiduel par rapport aux données, correspondant à établir un écart entre une grandeur estimée et une grandeur effectivement mesurée.

Ici, la grandeur estimée est:

$$\sum_m \sum_n G_{sm} X_{mn} G_{md} \alpha_n$$

pour la grandeur mesurée $M^{(0)}_{sd}$.

La grandeur estimée est :

$$\frac{\sum_m \sum_n G_{sm} X_{mn} G_{md} (Ts + Tsm + Tm + Tmd + Td)}{\sum_m \sum_n G_{sm} X_{mn} G_{md} \alpha_n}$$

pour la grandeur mesurée $M_{sd}^{(1)} / M_{sd}^{(0)}$

Au final, là encore, on sélectionnera dans chaque colonne, c'est-à-dire pour chaque fluorophore, la ligne m pour laquelle le paramètre, de base $\chi_{im}$ ou combiné Pm, donne la valeur minimum ou maximum, selon la nature de ce paramètre : comme déjà expliqué ci-dessus, on prendra pour chacun des paramètres $\chi_{1m}$, $\chi_{2m}$ la valeur maximum, et pour le paramètre $\chi_{3m}$ la valeur minimum ; et on applique ces principes à tout paramètre combiné qui résulte de ces paramètres de base.

La ligne m sélectionnée correspond à un voxel m, dans lequel le fluorophore n est probablement ou très probablement situé.

L'invention traite du cas de N (>1) fluorophores, N étant un nombre entier inconnu.

Physiquement, la situation est sensiblement la même que celle déjà décrite ci-dessus en combinaison avec la figure 6 et avec le cas précédent.

Mais on ignore le nombre de fluorophores, et donc le nombre de colonnes que la matrice X doit avoir.

On procède également par itération, et en utilisant les mêmes paramètres déjà présentés, $\chi_{1m}$, $\chi_{2m}$, $\chi_{3m}$ et éventuellement au moins un paramètre combiné $P_m$.

Ce procédé selon l'invention est maintenant expliquée en liaison avec la figure 8.

On initialise N, le nombre de fluorophore recherché, à 0 (étape S'0), puis on l'incrémente à 1 (étape S'1).

Puis (étape S'2), tous les éléments de la matrice sont initialisés à 0. On ne connaît que M (nombre de voxels discrétisant le milieu).

On initialise ensuite $N_{iter}$, le nombre d'itérations, à 0 (étape S'3), puis on l'incrémente à 1 (étape S'4). Lors de l'initialisation de $N_{iter}$ on put également déterminer le nombre maximum d'itérations que l'on souhaite réaliser.

Les étapes S'5 - S'11, sont identiques aux étapes S2 - S8. On se reportera donc à la description ci-dessus.

Si la corrélation est satisfaisante, le nombre de fluorophores est égal au nombre N courant et la matrice X est constituée des éléments précédemment déterminés (étape S'12).

C'est alors la fin du déroulement du procédé (étape S'13).

Si la corrélation n'est pas satisfaisante, on regarde si le nombre d'itérations et le nombre maximum $N_{iter}$ préa-lablement fixé (étape S'11) .

Si ce n'est pas le cas, on retourne à l'étape S'3 en incrémentant $N_{iter}$ d'une unité.

Si c'est le cas, dans la mesure où le test de corrélation a échoué, on incrémente le nombre de fluorophores d'une unité (retour à l'étape S'1).

La procédé continue jusqu'à ce que l'on ait déterminé une matrice pour laquelle le test de corrélation est satisfaisant (S'11 - S'13).

Au final, là encore, on sélectionnera dans chaque colonne, c'est-à-dire pour chaque fluorophore, la ligne m pour laquelle le paramètre, de base ou combiné, donne la valeur minimum ou maximum, selon la nature de ce paramètre : comme déjà expliqué ci-dessus, on prendra pour chacun des paramètres $\chi_{1m}$, $\chi_{2m}$ la valeur maximum, et pour le paramètre $\chi_{3m}$ la valeur minimum ; et on applique ces principes à tout paramètre combiné qui résulte de ces paramètres de base. La ligne m sélectionnée correspond à un voxel m, dans lequel le fluorophore n est probablement ou très probablement situé.

Une variante de ce procédé pour un nombre N de fluorophores inconnu peut être la suivante :

1) On prend N=1,
2) appliquer la reconstruction décrite ci-dessus en liaison avec la figure 7.pour N fluorophores,
3) test si l'attache aux données est satisfaisante,
4) si oui, fin ; Si non N=N+1, et on recommence à l'étape 2.

On donne ensuite un exemple avec deux fluorophores.

On applique le procédé de la figure 7.

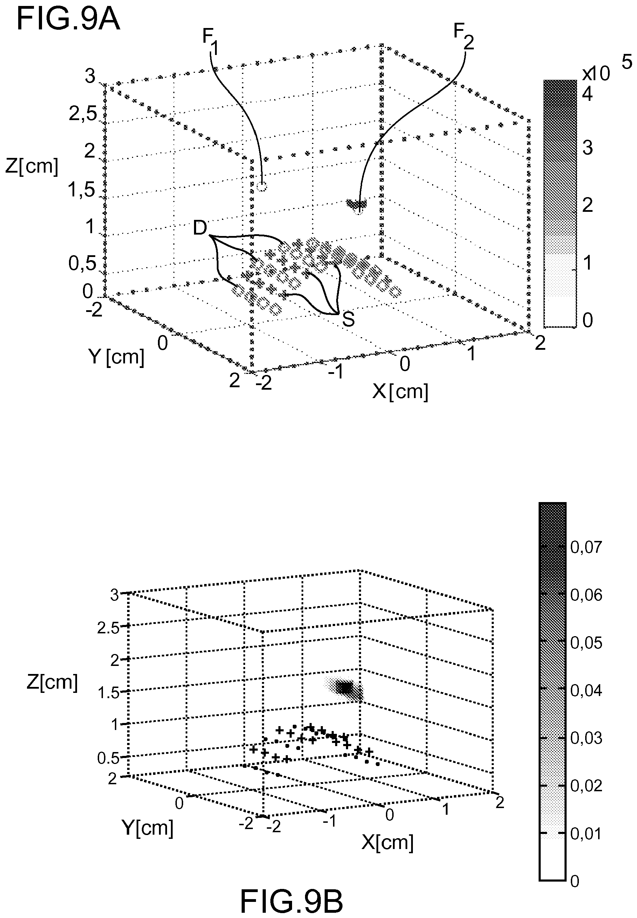

Les deux fluorophores sont désigné par $F_1$ et $F_2$ sur la figure 9A. Les sources S et les détecteurs D sont disposés sur une surface cylindrique, comme on le voir sur cette figure.

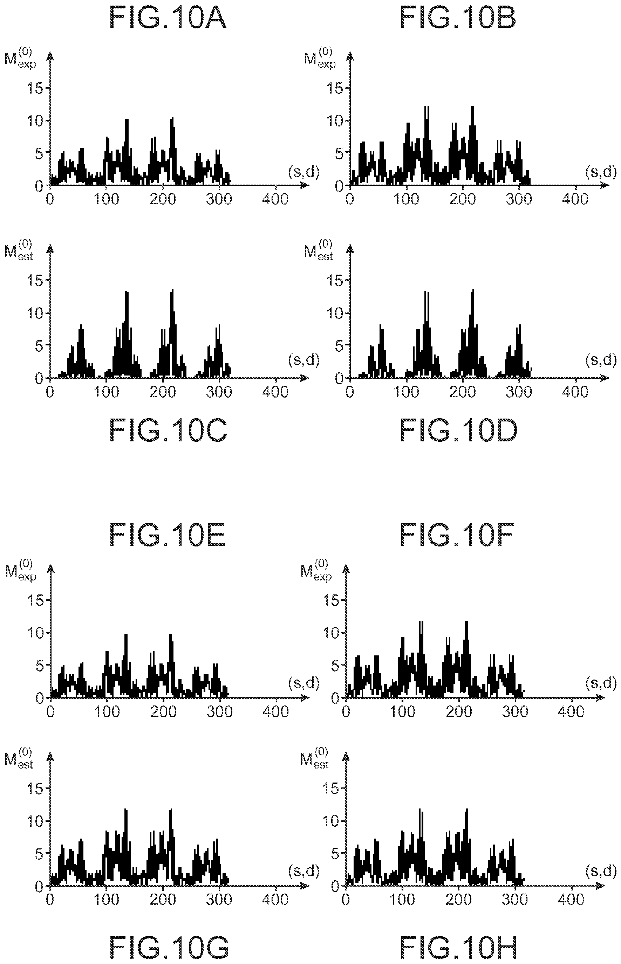

Lors de l'itération 1, on voit (figure 9B) qu'un des fluorophores est correctement reconstruit. Les figures 10A - 10D montrent des représentations de $M^{(0)}_{exp}$ (mesure de $M^{(0)}$, figure 10A), $M^{(1)}_{exp}$ (mesure de $M^{(1)}$, figure 10B) , $M^{(0)}_{est}$ (calcul de $M^{(0)}$, figure 10C), $M^{(1)}_{est}$ (calcul de $M^{(1)}$, figure 10D). Ces figures montrent que la convergence des estimations vers les données mesurées n'est pas satisfaisante.

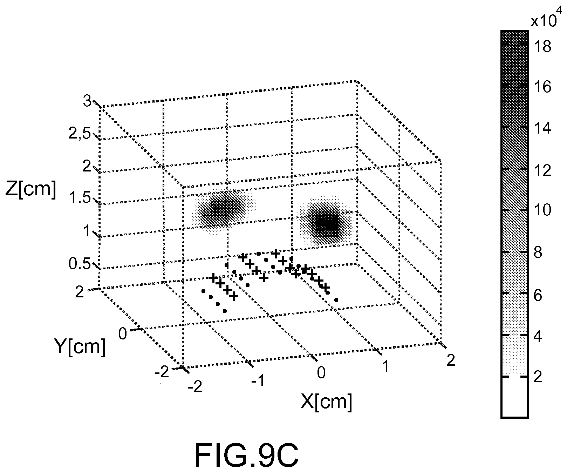

Lors de l'itération 2, on voit (figure 9C) que les deux fluorophores sont correctement reconstruits. Les figures 10E - 10H montrent des représentations de $M^{(0)}_{exp}$ (mesure de $M^{(0)}$, figure 10E), $M^{(1)}_{exp}$ (mesure de $M^{(1)}$, figure 10F), $M^{(0)}_{est}$ (calcul de $M^{(0)}$, figure 10G), $M^{(1)}_{est}$ (calcul de $M^{(1)}$, figure 10H). Ces figures montrent que la convergence des estimations vers les données mesurées est alors satisfaisante.

Un quatrième mode de réalisation non revendiqué, correspond à une généralisation du premier mode de réalisation. Dans ce dernier, on a vu qu'on pouvait établir des coefficients de base $\chi_{im}$ effectuant une somme, pour différentes acquisitions, chaque acquisition correpondant à un couple source-détecteur, d'une combinaison entre une grandeur établie à partir d'au moins un moment de $M_{sd}(t)$, et l'estimation de cette grandeur, cette estimation étant réalisée en considérant un fluorophore unique positionné dans le voxel m.

Ainsi, selon ce premier mode de réalisation, lors de la détermination de chaque coefficient de base $\chi_{im}$, on considérait qu'on se plaçait dans une configuration a priori selon laquelle le fluorophore était unique et situé dans le voxel m.

Selon ce quatrième mode de réalisation, on détermine également des coefficients de base $\chi_{im}$ similaires à ceux décrits dans le premier mode de réalisation, mais lors de la détermination des coefficients de base $\chi_{im}^N$, on se place dans une configuration a priori, selon laquelle on dispose de N fluorophores localisés dans N voxels différents. L'indice m correspond alors à une configuration, c'est-à-dire une répartition particulière de ces N fluorophores parmi les M voxels selon lesquels le milieu étudié a été discrétisé.

On comprend alors qu'il y a $M^N$ configurations possibles (au maximum). Ainsi, on pourra déterminer $M^N$ ièmes paramètres de base $\chi_{im}^N$.

Le premier mode de réalisation peut correspondre à un cas particulier de ce quatrième mode de réalisation, selon lequel N = 1 : il y a ainsi M paramètres $\chi_{im}^{N=1}$ déterminables, l'indice m correspondant à une répartition particulière du fluorophore unique parmi les M voxels du milieu : celle selon laquelle le fluorophore est situé dans le voxel m.

Ainsi, si l'on considère N fluorophores, N étant un entier naturel strictement positif, il est possible de déterminer au moins un paramètre de base $\chi_{im}^N$, égal à, pour au moins une acquisition $Msd(t)$ correspondant à un couple source détecteur, la combinaison d'une grandeur obtenue à partir d'au moins un moment de $Msd(t)$ avec une estimation de ladite grandeur, cette estimation étant réalisée en considérant que les N fluorophores sont répartis selon les M voxels du milieu selon une répartition donnée, ladite répartition étant indicée par le paramètre m.

Ainsi, chaque ième coefficient de base $\chi_{im}$ est obtenu en considérant une répartition donnée m des N fluorophores dans les M voxels. Comme rappelé ci-dessus, il y a donc $M^N$ répartitions possibles, correspondant à autant de ièmes coefficients de base possibles. Autrement dit, $1 \leq m \leq M^N$

Si l'on considère que la grandeur obtenue à partir d'au moins un moment de $M_{sd}(t)$ est un moment d'ordre 0 de $M_{sd}(t)$, noté $M_{sd}^{(0)}$, on peut alors établir un premier coefficient de base $\chi_{1m}^N$ tel que

$$\chi_{1m}^N = \min_{\alpha_1,...\alpha_N} \sum_{sd} \frac{\left(M_{sd}^{(0)} - M_{sd,m}^{theo\ 0}(\alpha_1,...\alpha_N)\right)^2}{\sigma^2\left(M_{sd}^0\right)}$$

avec :

$$M_{sd,m}^{theo\ 0}(\alpha_1,...\alpha_N) = \sum_{n=1}^N \alpha_n G_{sm_n} G_{m_n d}$$

On considère ici une configuration m correspond à N fluorophores répartis dans les voxels $m_n$, $1 \leq n \leq N$.

$G_{smn}$ (respectivement $G_{mnd}$) représentent les fonctions de transfert d'énergie entre la source et le voxel $m_n$ (respectivement le voxel $m_n$ et le détecteur d). Les coefficients $\alpha_n$, qui peuvent être considérés comme des intensités, sont obtenus, par l'opération de minimisation, de même que $X_{1m}$.

Chaque élément $(M_{sd}^0 - M_{sd,m}^{theo0}(\alpha_1,...\alpha_N))$ de la somme représente alors la différence, ou plus généralement la comparaison (il pourrait très bien s'agir d'un ratio) entre :

- la valeur de l'intensité mesurée $M_{sd}^0$ pour le couple (source, détecteur) sélectionné,
- et une estimation du moment $M_{sd}^0$ d'ordre zéro, obtenue à l'aide d'une part des fonctions de Green $G_{smn}$ et $G_{mnd}$, qui peuvent être estimées de la manière déjà décrite ci-dessus, et d'autre part d'un ensemble de coefficients $\alpha_n$ dont chacun est affecté à un fluorophore et qui représente l'intensité d'émission de la fluorescence par celui-ci.

Ce premier paramètre de base $\chi_{1m}^N$ est minimum pour les configurations m les plus proches de la répartition réelle des N fluorophores dans le milieu étudié.

Si l'on considère que la grandeur obtenue à partir d'au moins un moment de $M_{sd}(t)$ est un moment normé d'ordre 1 de $M_{sd}(t)$, noté $M_{1d}^{(1)} / M_{sd}^{(0)}$, pouvant être également noté $T_{sd}$, auquel on peut, éventuellement, retrancher les grandeurs temporelles connues Ts (temps moyen de la source), Td (temps de réponse du détecteur) et $\sigma$ (durée de la fluorescence), on peut alors établir un deuxième paramètre de base $\chi_{2m}$ tel que

$$\chi_{2m}{}^{N} = \min_{\alpha'_1,\dots\alpha'_N} \sum_{sd} \frac{\left((T_{sd} - T_s - \sigma - T_d) - (T_{sd,m}^{theo}(\alpha'_1,\dots\alpha'_N))\right)^2}{\sigma^2\left(T_{sd}\right)}$$

Avec :

$$T_{sd,m}^{theo}(\alpha'_1,\dots\alpha'_N) = \frac{\sum\limits_{n=1}^{N} \alpha'_n\, G_{sm_n} G_{m_n d}\left(T_{sm_n} + T_{m_n d}\right)}{\sum\limits_{n=1}^{N} \alpha'_n\, G_{sm_n} G_{m_n d}}$$

On considère ici une configuration m correspond à N fluorophores répartis dans les voxels $m_n$, $1 \le n < N$.

$G_{smn}$ (respectivement $G_{mnd}$) représentent les fonctions de transfert d'énergie entre la source et le voxel $m_n$ (respectivement le voxel $m_n$ et le détecteur d) . $T^{theo}{}_{sd}$ ($\alpha1$, $\dots\alpha N$) ainsi défini correspond bien à une estimation de $T_{sd}$ - $T_s$ - $T_d$ - $\tau$.

On voit que $\chi_{2m}{}^N$ est la différence (ou un résidu, et plus généralement une comparaison, cette comparaison pouvant être également réalisée par un ratio) entre, pour au moins une mesure, entre une grandeur constituée par le temps de vol moyen source-détecteur mesuré (moment normé d'ordre 1 de M(t)), de préférence, mais pas nécessairement, diminué de grandeurs temporelles connues ($T_s$, $T_d$, $\tau$), et cette même grandeur modélisée à l'aide d'une contribution de l'ensembles des fluorophores répartis selon la configuration m, par le biais des coefficients $\alpha'_i$ dont chacun est affecté à un fluorophore et qui représente l'intensité d'émission de la fluorescence par celui-ci.

Les $\alpha'_n$ sont obtenus, par l'opération de minimisation, de même que $\chi_{2m}{}^N$. Ce dernier, appelé aussi critère de résidu des mesures de temps de vol, est lui aussi petit pour des configurations m correspondant à la répartition réelle des N fluorophores dans le milieu.

De façon préférentielle, et comme c'est le cas dans la formule ci-dessus, le paramètre $\chi_{2m}{}^N$ peut être normé par une grandeur statistique relative à la distribution de $T_{sd}$. Cette grandeur statistique joue le rôle d'indicateur de confiance, comme déjà expliqué ci-dessus pour $\chi_{2m}$. On a choisi ici $\sigma^2$ ($T_{sd}$), mais une autre grandeur statistique (par exemple écart type ou variance) pourrait être choisie, pour indiquer la confiance qu'on attribue à une distribution, mais comme on l'a vu précédemment, la normalisation par le coefficient $\sigma^2$ ($T_{sd}$) est préférée.

D'autres paramètres de base obtenus à partir d'autres moments de Msd(t) peuvent être constitués. Cependant, les paramètres de base $\chi_{1m}{}^N$ et $\chi_{2m}{}^N$ définis sont particulièrement pertinents.

A partir d'un ou plusieurs paramètres de base $\chi_{1m}{}^N$ constitués, il est possible de définir un paramètre combiné $P_m{}^N$, combinant lesdits paramètres de base $\chi_{im}{}^N$. Par combinaison, on rappelle qu'on entend toute opération arithmétique linéaire ou non, les opérations les plus simples étant la multiplication, l'addition ou les ratios.

Selon un mode de réalisation préféré, en utilisant les paramètres $\chi_{1m}{}^N$ et $\chi_{2m}{}^N$ précédemment obtenus, on peut ainsi constituer un paramètre $P_m{}^N$ tel que $P_m{}^N = \chi_{1m}{}^N + \chi_{2m}{}^N$. Ce coefficient $P_m{}^N$ combiné sera minimal lorsque la répartition des N fluorophores d'indice m sera la plus proche de la réalité.

Lorsque $P_m{}^N$ est une combinaison des paramètres de base $\chi_{1m}{}^N$ et $\chi_{2m}{}^N$, on peut choisir un ensemble de coefficients $\alpha_n$ (1<n<N) ou $\alpha'_n$ (1<n<N) minimisant soit $\chi_{1m}{}^N$, soit $\chi_{2m}{}^N$, soit $P_m{}^N$.

Il est alors possible d'obtenir un coefficient combiné $P_m{}^N$ pour tout ou partie des $M^N$ répartitions possibles des N flurophores dans les M voxels, une configuration m correspondant à un positionnement des N fluorophores selon les voxels $m_1\dots m_N$. On détermine ensuite les répartitions dont le coefficient combiné $P_m{}^N$ est inférieur à une valeur limite $P_m{}^N$ limite déterminée par l'opérateur. Cette valeur $P_m{}^N$ limite peut par exemple être égale à la plus grande valeur $P_m{}^N$ obtenue divisée par deux.

On peut également déterminer un paramètre de base $\chi_{4m}{}^N$, tel que :

$$\chi_{4m}{}^{N} = \min_{\alpha_1,\dots\alpha_N}\left[ \sum_{sd} \frac{\left(M_{sd}{}^{(0)} - M_{sd,m}^{theo\ 0}(\alpha_1,\dots\alpha_N)\right)^2}{\sigma^2\left(M_{sd}{}^0\right)} + \sum_{sd} \frac{\left((T_{sd} - T_s - \sigma - T_d) - (T_{sd,m}^{theo}(\alpha_1,\dots\alpha_N))\right)^2}{\sigma^2\left(T_{sd}\right)} \right]$$

avec :

$$M_{sd,m}^{theo\ 0}(\alpha_1,...\alpha_N) = \sum_{n=1}^{N} \alpha_n G_{sm_n} G_{m_n d}$$

et :

$$T_{sd,m}^{theo}(\alpha_1,...\alpha_N) = \frac{\sum_{n=1}^{N} \alpha_n G_{sm_n} G_{m_n d}\left(T_{sm_n} + T_{m_n d}\right)}{\sum_{n=1}^{N} \alpha_n G_{sm_n} G_{m_n d}}$$

On retrouve ici un calcul d'un paramètre de base $\chi_{4,m}^N$, combinant, pour au moins une desdites distributions temporelles $M_{sd}(t)$, au moins une grandeur (il s'agit ici des deux grandeurs $M_{sd}^0$ et $T_{sd}$) obtenue à partir d'au moins un moment de ladite distribution, et au moins une estimation de ces grandeurs (il s'agit ici de $M_{sd,m}^{theo0}$ et $T_{sd,m}^{theo0}$), ces estimations étant réalisées en considérant qu'on dispose de N fluorophores, chacun occupant alors un voxel du milieu selon une répartition m.

On peut déterminer $P_m^N$ à partir d'un seul paramètre de base $\chi_{im}^N$, le paramètre $P_m^N$ pouvant même être égal à un critère de base $\chi_{im}^N$. Par exemple, on peut déterminer $P_m^N$ de telle sorte que

$$P_m^N = \chi_{4m}^N$$

Lorsque N=1, on retrouve bien $\chi_{1m}^{N=1} = \chi_{1m}^{N=1}$ et $\chi_2 m^{N=1} = \chi_{2m}$, $\chi_{1m}$ et $\chi_{2m}$ étant tels que définis dans la description du premier mode de réalisation.

L'invention s'applique avantageusement à la localisation de tumeur dans le cas de la prostate, mais peut s'appliquer à d'autres organes, notamment testicules, sein, cerveau....

Ainsi, on a pu à l'aide de l'invention, reconstruire la fluorescence de capillaires enfouis à 1cm ou 2cm.

Dans cet exemple, la prise en compte de la précision de la mesure temporelle a permis de rejeter certaines mesures aberrantes ; en effet, comme les temps moyens sont obtenus par division de M1 par M0, ce rapport devient très imprécis lorsqu'il y a très peu de signal.

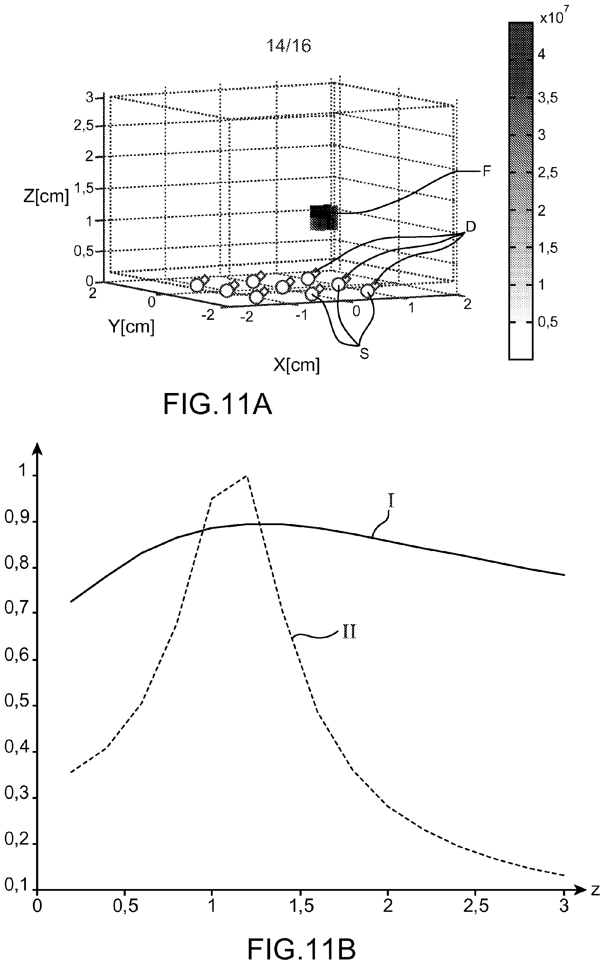

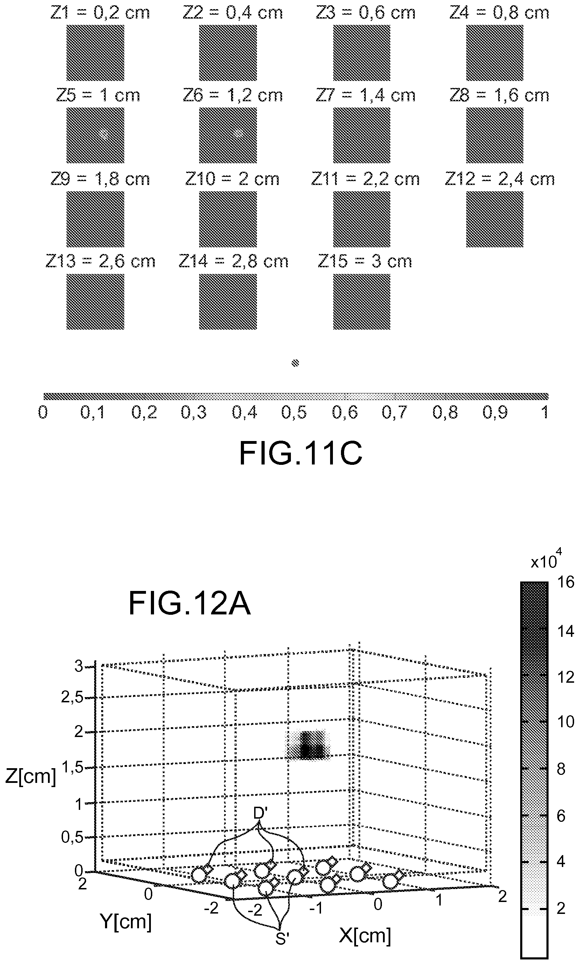

Les Figure 11A - 11C et 12A - 12C illustrent des résultats obtenus.

La figure 11A représente une configuration avec un capillaire à environ 1cm de profondeur, avec 9 sources S et 9 détecteurs D disposés dans le plan z = 0. La valeur de $\tau$ est 0,95 ns. Le critère $\chi_{2m}$ est ici utilisé, avec l'approximation $T_s = T_d = 0$. La profondeur trouvée est de z=l,lcm, pour x=0,7 cm et y = 0 cm.

Le graphique de la figure 11B montre que des mesures basées sur les intensités seules (courbe I) donnent des résultats sans précision suivant z, tandis que celles basées sur les résultats temporels donnent des résultats précis suivant z (courbe II), ce que confirme la figure 11C.

La figure 12A représente une configuration avec un capillaire à environ 2cm de profondeur, avec 9 sources S' et 9 détecteurs D' disposés dans le plan z = 0. La valeur de $\tau$ est 0,95 ns. Le critère $\chi_{2m}$ est ici utilisé, avec l'approximation $T_s = T_d = 0$. La profondeur trouvée est de z=1,9cm, pour x=0,2 cm et y = 0 cm..

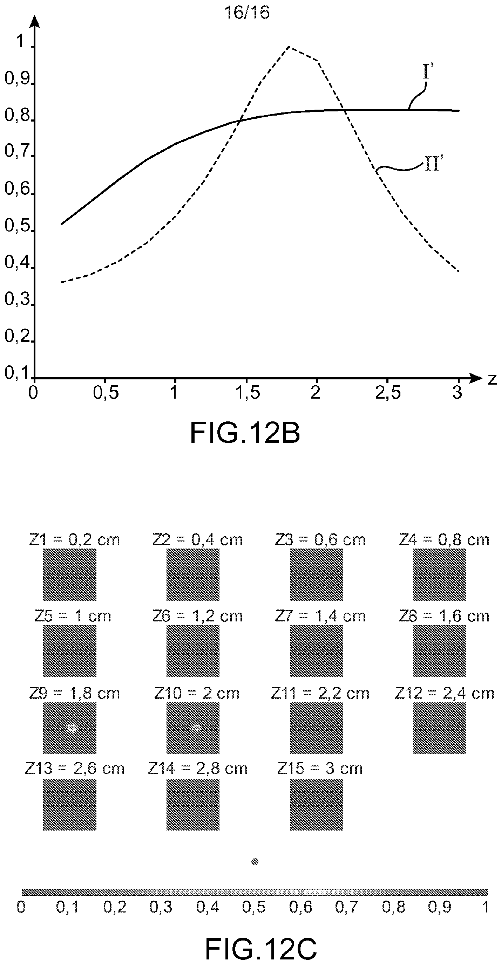

Le graphique de la figure 12B montre que des mesures basées sur les intensités seules (courbe I') donnent des résultats sans précision suivant z, tandis que celles basées sur les résultats temporels donnent des résultats précis suivant z (courbe II'), ce que confirme la figure 12C.

Dans un procédé selon l'invention, le milieu environnant le fluorophore ou l'absorbeur peut être de type infini ou de type semi-infini ou en forme une tranche, limitée par deux surfaces parallèles, ou est de forme quelconque, sa surface extérieure étant discrétisée en une série de plans.

Un procédé selon l'invention est particulièrement bien adapté au cas où les sources de rayonnement et les détecteurs sont en géométrie de réflexion.

Le coefficient de base $\chi_{1m}^N$ peut être tel que :

$$\chi_{1m}^N = \min_{\alpha_1,...\alpha_N} \sum_{sd} \frac{\left(M_{sd}^{(0)} - M_{sdm}^{theo\ 0}(\alpha_1,...\alpha_N)\right)^2}{\sigma^2\left(M_{sd}^0\right)}$$

avec :

$$M_{sd,m}^{theo\ 0}(\alpha_1,...\alpha_N) = \sum_{n=1}^{N} \alpha_n G_{sm_n} G_{m_n d}$$

- une configuration m correspondant à N fluorophores répartis dans les voxels $m_n$, $1 \leq n \leq N$,
- $G_{smn}$ (respectivement $G_{mnd}$) représentant les fonctions de transfert d'énergie entre la source et le voxel $m_n$ (respectivement le voxel $m_n$ et le détecteur d). Les coefficients $\alpha_n$, qui peuvent être considérés comme des intensités, sont obtenus, par l'opération de minimisation, de même que $X_{1m}$;
- Chaque élément ($M_{sd}^0 - M_{sd,m}^{theo0}(\alpha_1,...\alpha_N)$) de la somme représentant alors la différence entre :

    - la valeur de l'intensité mesurée $M_{sd}^0$ pour le couple (source, détecteur) sélectionné,
    - et une estimation du moment $M_{sd}^0$ d'ordre zéro, obtenue à l'aide d'une part des fonctions de Green $G_{smn}$ et $G_{mnd}$, , et d'autre part d'un ensemble de coefficients $\alpha_n$ dont chacun est affecté à un fluorophore et qui représente l'intensité d'émission de la fluorescence par celui-ci.

Le deuxième paramètre de base $\chi_{2m}^N$ peut être tel que :

$$\chi_{2m}^N = \min_{\alpha'_1,...\alpha'_N} \sum_{sd} \frac{\left((T_{sd} - T_s - \sigma - T_d) - (T_{sd,m}^{theo}(\alpha'_1,...\alpha'_N))\right)^2}{\sigma^2(T_{sd})}$$

Avec :

$$T_{sd,m}^{theo}(\alpha'_1,...\alpha'_N) = \frac{\sum_{n=1}^{N} \alpha'_n G_{sm_n} G_{m_n d}(T_{sm_n} + T_{m_n d})}{\sum_{n=1}^{N} \alpha'_n G_{sm_n} G_{m_n d}}$$

Ts, Td et $\tau$ représentent respectivement les temps de réponse de la source, du détecteur et la durée de fluorescence du fluorophore, $Tsm_n$ et $Tm_n d$ représentent les temps de vol respectifs entre la source et le fluorophore localisé dans le voxel $m_n$, et entre le fluorophore localisé dans le voxel $m_n$ et le détecteur, le coefficient $\sigma^2(Tsd)$ correspondant à une estimation de la variance de la distribution Tsd, $T_{sd,m}^{theo}(\alpha'1...\alpha'N)$ représentant alors une estimation du moment normé d'ordre 1 de la fonction $M_{sd}(t)$, les coefficients $\alpha'_N$ étant obtenus par l'opération de minimisation.Un dispositif selon l'invention peut comporter des moyens pour déterminer un paramètre combiné $P_m^N$, égal à la somme ou au produit des coefficients de base $\chi_{1,m}^N$, et $\chi_{2,m}^N$. Les paramètres combinés $P_m^{N=1}$ de plus faible valeur peuvent correspondre alors aux répartitions des fluorophores les plus proches de la réalité. Dans un dispositif selon l'invention, les sources de rayonnement et les détecteurs sont de préférence en géométrie de réflexion.

## Revendications

1. Procédé de localisation d'au moins N (N>1) fluorophores (F1, F2), N étant un nombre inconnu, dans un milieu diffusant à l'aide d'au moins une source ($S_1$ - $S_4$, $L_1$-$L_4$,) de rayonnement pulsée apte à émettre un rayonnement d'excitation de ces fluorophores et d'au moins un détecteur ($D_1$ - $D_5$) apte à mesurer un signal de fluorescence émis par ces fluorophores comportant:

    - l'éclairement du milieu par une source de rayonnement,
    - la détection, par au moins un détecteur, du signal, d'intensité mesurée $M_{sd}^0$, produit par le milieu à la longueur d'onde de fluorescence,
    - pour au moins un couple source-détecteur, la réalisation d'une distribution temporelle $M_{sd}(t)$ du signal reçu par le détecteur,
    - le milieu diffusant étant discrétisé en M voxels, en considérant qu'on dispose d'un nombre N courant de fluorophores, chaque fluorophore occupant un voxel parmi les M voxels du milieu, selon une répartition m,

- pour différentes répartitions m, le calcul d'au moins un paramètre de base $\chi^N_{i,m}$, combinant, pour au moins une desdites distributions temporelles $M_{sd}(t)$, au moins une grandeur obtenue à partir d'au moins un moment de ladite distribution, et au moins une estimation de cette grandeur ; l'au moins un paramètre de base donnant une information sur la localisation des fluorophores;
- procédé dans lequel on réalise les étapes suivantes pour au moins un paramètre de base:

  * une étape de calcul d'une corrélation $(S_8, S'_{11})$ entre l'intensité mesurée et l'ensemble des contributions à l'intensité de l'ensemble des fluorophores,
  * une étape $(S'_1)$ d'incrémentation du nombre N courant de fluorophores, si le calcul d'une corrélation n'est pas satisfaisant.

2. Procédé selon la revendication 1, comportant également la détermination d'un paramètre combiné $P_m^N$, combinant au moins deux paramètres de base $\chi^N_{i,m}$, par exemple égal à la somme ou au produit de deux paramètres de base $\chi^{N=1}_{1,m}$, et $\chi^{N=1}_{2,m}$, les paramètres combinés $P_m^{N=1}$ de plus faible valeur correspondant alors aux répartitions des fluorophores les plus proches de la réalité.

3. Procédé selon l'une des revendications précédentes, l'un des paramètres de base $\chi^N_{2,m}$ comportant une comparaison entre le moment normé d'ordre 1 de ladite distribution temporelle, corrigée de l'estimation de la contribution au temps de vol des fluorophores déjà localisés, et la modélisation de ce moment normé d'ordre 1 ou une comparaison entre le moment d'ordre 0 de ladite distribution temporelle, corrigée de l'estimation de la contribution à l'intensité des fluorophores déjà localisés, et la modélisation de ce moment d'ordre 0.

4. Procédé selon l'une des revendications précédentes, comportant la détermination d'au moins un des paramètres de base suivant :

   - la détermination d'un premier paramètre de base $\chi^N_{1,m}$, qui est la somme, pour tous les couples (source, détecteur), des différences entre la valeur de l'intensité mesurée $M_{sd}^0$ pour chaque couple (source, détecteur), corrigée de l'estimation de la contribution à l'intensité des fluorophores déjà localisés, et une estimation du moment d'ordre zéro, pour chaque couple (source, détecteur), obtenue à l'aide des fonctions de Green $G_{smn}$ et $G_{mnd}$, pour la source et le détecteur de chaque couple, cette estimation étant réalisée en considérant que les N fluorophores sont répartis dans les voxels du milieu selon la configuration m, selon laquelle les fluorophores sont répartis dans les voxels $m_n$ ;
   - la détermination d'un second paramètre de base $\chi^N_{2,m}$, qui est la somme, pour tous les couples (source, détecteur), des différences entre, pour chaque couple source -détecteur, le temps de vol moyen source-détecteur mesuré (moment normé d'ordre 1 de M(t)), corrigé de grandeurs temporelles connues relatives à la source, au détecteur et au fluorophore, et corrigé de l'estimation de la contribution au temps de vol des fluorophores déjà localisés, et l'estimation de ce temps de vol corrigé, cette estimation étant réalisée en considérant que les N fluorophores sont répartis dans les voxels du milieu selon la configuration m.

5. Procédé selon la revendication 4, selon lequel le coefficient de base $\chi_{1m}^N$ est tel que :

$$\chi_{1m}^N = \min_{\alpha_1,\ldots\alpha_N} \sum_{sd} \frac{\left(M'^{(0)}_{sd} - M^{theo\ 0}_{sd,m}(\alpha_1,\ldots\alpha_N)\right)^2}{\sigma^2\left(M_{sd}^0\right)}$$

avec :

$$M^{theo\ 0}_{sd,m}(\alpha_1,\ldots\alpha_N) = \sum_{n=1}^{N} \alpha_n G_{sm_n} G_{m_n d}$$

   - une configuration m correspondant à N fluorophores répartis dans les voxels $m_n$, $1 \leq n \leq N$,
   - $G_{smn}$ (respectivement $G_{mnd}$) représentant les fonctions de transfert d'énergie entre la source et le voxel $m_n$ (respectivement le voxel $m_n$ et le détecteur d), les coefficients $\alpha_n$, résultant de l'opération de minimisation, de même que $\chi_{1m}$;
   - Chaque élément $(M'^0_{sd} - M^{theo0}_{sd,m}(\alpha_1,\ldots \alpha_N))$ de la somme représentant alors la différence entre :

- la valeur $M'^0_{sd}$ de l'intensité mesurée $M^0_{sd}$ pour le couple (source, détecteur) sélectionné, corrigée de l'estimation de la contribution à l'intensité des fluorophores déjà localisés,
- et une estimation du moment $M^0_{sd}$ d'ordre zéro, obtenue à l'aide d'une part des fonctions de Green $G_{smn}$ et $G_{mnd}$, et d'autre part d'un ensemble de coefficients $\alpha_n$ dont chacun est affecté à un fluorophore et qui représente l'intensité d'émission de la fluorescence par celui-ci,

et le deuxième paramètre de base $\chi_{2m}^N$ pouvant être tel que :

$$\chi_{2m}^{\ N} = \min_{\alpha'_1,...\alpha'_N} \sum_{sd} \frac{\left(\left((M'^{(1)}_{sd}/M'^{(0)}_{sd}) - T_s - \sigma - T_d\right) - (T^{theo}_{sd,m}(\alpha'_1,...\alpha'_N))\right)^2}{\sigma^2\left(T_{sd}\right)}$$

Avec :

$$T^{theo}_{sd,m}(\alpha'_1,...\alpha'_N) = \frac{\sum_{n=1}^{N} \alpha'_n\, G_{sm_n} G_{m_n d}\left(T_{sm_n} + T_{m_n d}\right)}{\sum_{n=1}^{N} \alpha'_n\, G_{sm_n} G_{m_n d}}$$

Ts, Td et $\tau$ représentant respectivement les temps de réponse de la source, du détecteur et la durée de fluorescence du fluorophore, $Tsm_n$ et $Tm_nd$ représentant les temps de vol respectifs entre la source et le fluorophore localisé dans le voxel $m_n$, et entre le fluorophore localisé dans le voxel $m_n$ et le détecteur, le coefficient $\sigma^2$ (Tsd) correspondant à une estimation de la variance la distribution Tsd, $T^{theo}_{sd}$ ($\alpha'1...\alpha'N$) représentant alors une estimation du moment normé d'ordre 1 de la fonction $M_{sd}(t)$, les coefficients $\alpha'_N$ étant obtenus par l'opération de minimisation, $M'^{(1)}_{sd}$ étant le moment d'ordre 1, corrigé de l'estimation de la contribution au temps de vol des fluorophores déjà localisés.

6. Dispositif de localisation d'au moins moins N (N>1) fluorophores ($F_1$, $F_2$), N étant un nombre inconnu, dans un milieu diffusant, comportant au moins une source ($S_1$ - $S_4$, $L_1$-$L_4$,) de rayonnement pulsée apte à émettre un rayonnement d'excitation de ces fluorophores et au moins un détecteur ($D_1$ - $D_5$) apte à mesurer un signal de fluorescence, d'intensité mesurée $M^0_{sd}$, émis par ces fluorophores comportant:

- des moyens (26) pour réaliser, pour au moins un couple source-détecteur, une distribution temporelle $M_{sd}(t)$ du signal reçu par le détecteur,
- des moyens (26) pour réaliser un maillage (M) du volume en mailles élémentaires, ou voxels, et pour répartir un nombre N courant de fluorophores dans le milieu, chaque fluorophore étant réparti dans un des M voxels du milieu selon une répartition m,
- des moyens (26) pour calculer, pour différentes répartitions m, au moins un paramètre de base $\chi^N_{i,m}$, combinant, pour au moins une desdites distributions temporelles $M_{sd}(t)$, au moins une grandeur obtenue à partir d'au moins un moment de ladite distribution, et au moins une estimation de cette grandeur ; l'au moins un paramètre de base donnant une information sur la localisation des fluorophores;
- ce dispositif comportant en outre des moyens pour, pour au moins un paramètre de base:

  * calculer une corrélation entre l'intensité mesurée et l'ensemble des contributions à l'intensité de l'ensemble des fluorophores ;
  * incrémenter le nombre N courant de fluorophores si le calcul d'une corrélation n'est pas satisfaisant.

7. Dispositif selon la revendication 6, comportant en outre des moyens (26) pour déterminer un paramètre combiné $P_m^N$, combinant au moins deux paramètres de base $\chi^N_{i,m}$, par exemple égal à la somme ou au produit des paramètres de base $\chi^N_{1,m}$, et $\chi^N_{2,m}$.

8. Dispositif selon l'une des revendications 6 ou 7, les moyens (26) pour calculer au moins un paramètre comportant des moyens pour déterminer un paramètre $\chi^N_{2,m}$ comportant la comparaison entre le moment normé d'ordre 1 de ladite distribution temporelle, corrigée de l'estimation de la contribution au temps de vol des fluorophores déjà localisés, et la modélisation de ce moment normé d'ordre 1 ou entre le moment d'ordre 0 de ladite distribution temporelle, corrigée de l'estimation de la contribution à l'intensité des fluorophores déjà localisés, et la modélisation de ce moment d'ordre 0.

**9.** Dispositif selon l'une des revendications 6 à 8, les moyens (26) pour calculer au moins un paramètre comportant des moyens pour :

- déterminer un premier paramètre de base $\chi^N_{1,m}$, qui est la somme, pour tous les couples (source, détecteur), des différences entre la valeur de l'intensité mesurée $M_{sd}^0$ pour chaque couple (source, détecteur), corrigée de l'estimation de la contribution à l'intensité des fluorophores déjà localisés, et une estimation du moment d'ordre zéro, pour chaque couple (source, détecteur), obtenue à l'aide des fonctions de Green $G_{smn}$ et $G_{mnd}$, pour la source et le détecteur de chaque couple, cette estimation étant réalisée en considérant que les N fluorophores sont répartis dans les voxels du milieu selon la configuration m, selon laquelle les fluorophores sont répartis dans les voxels $m_n$ ;
- déterminer un second paramètre de base $\chi^N_{2,m}$, qui est la somme, pour tous les couples (source, détecteur), des différences entre, pour chaque couple source -détecteur, le temps de vol moyen source-détecteur mesuré (moment normé d'ordre 1 de M(t)), corrigé de grandeurs temporelles connues relatives à la source, au détecteur et au fluorophore, et corrigé de l'estimation de la contribution au temps de vol des fluorophores déjà localisés, et l'estimation de ce temps de vol corrigé, cette estimation étant réalisée en considérant que les N fluorophores sont répartis dans les voxels du milieu selon la configuration m.

## Patentansprüche

**1.** Verfahren zum Lokalisieren von mindestens N (N > 1) Fluorophoren (F1, F2), wobei N eine unbekannte Zahl ist, in einem streuenden Medium mittels zumindest einer gepulsten Strahlungsquelle ($S_1$ - $S_4$, $L_1$ - $L_4$), die dazu geeignet ist, eine Strahlung zum Anregen dieser Fluorophore zu emittieren, und mittels zumindest eines Detektors ($D_1$ - $D_5$), der dazu geeignet ist, ein von diesen Fluorophoren emittiertes Fluoreszenzsignal zu messen, umfassend:

- Beleuchten des Mediums mit einer Strahlungsquelle,
- Detektion des Signals mit gemessener Intensität $M^0_{sd}$, das von dem Medium bei der Fluoreszenzwellenlänge erzeugt wird, durch zumindest einen Detektor,
- für zumindest ein Quelle-Detektor-Paar, Erstellen einer zeitlichen Verteilung $M_{sd}(t)$ des von dem Detektor empfangenen Signals,
- wobei das streuende Medium in N Voxel diskretisiert wird unter der Maßgabe, dass eine aktuelle Anzahl N von Fluorophoren vorliegt, wobei jeder Fluorophor ein Voxel unter den N Voxeln des Mediums gemäß einer Aufteilung m belegt,
- für verschiedene Aufteilungen m das Berechnen zumindest eines Basisparameters $\chi^N_{i,m}$, der für zumindest eine der zeitlichen Verteilungen $M_{sd}(t)$ zumindest eine Größe, die aus zumindest einem Moment der Verteilung erhalten wird, und zumindest eine Schätzung dieser Größe kombiniert, der zumindest eine Basisparameter gibt Auskunft über den Standort von Fluorophoren;
- bei welchem Verfahren ferner die nachstehenden Schritte erfolgen:

  * ein Schritt des Berechnens einer Korrelation ($S_8$, $S'_{11}$) zwischen der gemessenen Intensität und der gesamten Beteiligung bei der Intensität der gesamten Fluorophore,
  * ein Schritt ($S'_1$) des Inkrementierens der aktuellen Anzahl N an Fluorophoren, wenn das Berechnen einer Korrelation nicht zufriedenstellend ist.

**2.** Verfahren nach Anspruch 1, ferner umfassend das Ermitteln eines kombinierten Parameters $P_m^N$, der zumindest zwei Basisparameter $\chi^N_{i,m}$ kombiniert und beispielsweise gleich der Summe oder dem Produkt zweier Basisparameter $\chi^{N=1}_{1,m}$ und $\chi^{N=1}_{2,m}$ ist, wobei die kombinierten Parameter $P_m^{N=1}$ mit geringerem Wert dann den Aufteilungen der Fluorophore entsprechen, die der Realität am nächsten sind.

**3.** Verfahren nach einem der vorangehenden Ansprüche, wobei einer der Basisparameter $\chi^N_{2,m}$ einen Vergleich zwischen dem normierten Moment der Ordnung 1 der zeitlichen Verteilung, korrigiert um die Schätzung der Beteiligung an der Laufzeit der bereits lokalisierten Fluorophore, und der Modellierung dieses normierten Moments der Ordnung 1, oder einen Vergleich zwischen dem Moment der Ordnung 0 der zeitlichen Verteilung, korrigiert um die Schätzung der Beteiligung an der Intensität der bereits lokalisierten Fluorophore, und der Modellierung dieses Moments der Ordnung 0 enthält.

**4.** Verfahren nach einem der vorangehenden Ansprüche, umfassend das Ermitteln zumindest eines der nachstehenden Basisparameter:

- das Ermitteln eines ersten Basisparameters $\chi^N_{1,m}$, der für alle Paare (Quelle, Detektor) die Summe der Differenzen zwischen dem Wert der gemessenen Intensität $M_{sd}^0$ für jedes Paar (Quelle, Detektor), korrigiert um die Schätzung der Beteiligung an der Intensität der bereits lokalisierten Fluorophore, und einer Schätzung des Moments der Ordnung null für jedes Paar (Quelle, Detektor) ist, die mit Hilfe der Greenschen Funktionen $G_{smn}$ und $G_{mnd}$ für die Quelle und den Detektor eines jeden Paares erhalten wird, wobei diese Schätzung unter der Maßgabe erfolgt, dass die N Fluorophore in den Voxeln des Mediums gemäß der Konfiguration m aufgeteilt sind, wonach die Fluorophore in den Voxeln $m_n$ aufgeteilt sind;

- das Ermitteln eines zweiten Basisparameters $\chi^N_{2,m}$, der für alle Paare (Quelle, Detektor) die Summe der Differenzen für jedes Paar Quelle-Detektor zwischen der gemessenen mittleren Laufzeit Quelle-Detektor (normierter Moment der Ordnung 1 von M(t)), korrigiert um bekannte zeitliche Größen bezogen auf die Quelle, auf den Detektor und auf den Fluorophor, und korrigiert um die Schätzung der Beteiligung an der Laufzeit der bereits lokalisierten Fluorophore, und der Schätzung dieser korrigierten Laufzeit ist, wobei diese Schätzung unter der Maßgabe erfolgt, dass die N Fluorophore in den Voxeln des Mediums gemäß der Konfiguration m aufgeteilt sind.

**5.** Verfahren nach Anspruch 4, wobei der Basiskoeffizient $\chi_{1m}^N$ derart ist, dass

$$\chi_{1m}^{\ N} = \min_{\alpha_1,...\alpha_N} \sum_{sd} \frac{\left(M'^{(0)}_{sd} - M^{theo\ 0}_{sd,m}(\alpha_1,...\alpha_N)\right)^2}{\sigma^2\left(M_{sd}^{\ 0}\right)}$$

mit:

$$M^{theo\ 0}_{sd,m}(\alpha_1,...\alpha_N) = \sum_{n=1}^{N} \alpha_n G_{sm_n} G_{m_n d}$$

wobei

- eine Konfiguration m N Fluorophoren entspricht, die in den Voxeln $m_n$ aufgeteilt sind, $1 \leq n \leq N$,
- $G_{smn}$ (bzw. $G_{mnd}$) die Funktionen des Energietransfers zwischen der Quelle und dem Voxel $m_n$ (bzw. dem Voxel $m_n$ und dem Detektor d), die Koeffizienten $\alpha_n$, die aus der Minimierungsoperation resultieren, ebenso wie $\chi_{1m}$, darstellen;
- jedes Element ($M'^0_{sd} - M^{theo0}_{sd,m}(\alpha_1, ... \alpha_N)$) der Summe dann die Differenz darstellt zwischen:

  - dem Wert $M'^0_{sd}$ der gemessenen Intensität $M_{sd}^0$ für das ausgewählte Paar (Quelle, Detektor), korrigiert um die Schätzung der Beteiligung an der Intensität der bereits lokalisierten Fluorophore,
  - und einer Schätzung des Moments $M_{sd}^0$ der Ordnung null, die einerseits mit Hilfe der Greenschen Funktionen $G_{smn}$ und $G_{mnd}$ und andererseits mit Hilfe einer Gesamtheit von Koeffizienten $\alpha_n$ erhalten wird, von denen jeder einem Fluorophor zugordnet ist und die Fluoreszenzemissionsintensität von diesem darstellt,

und der zweite Basisparameter $\chi_{2m}^N$ derart sein kann, dass

$$\chi_{2m}^{\ N} = \min_{\alpha'_1,...\alpha'_N} \sum_{sd} \frac{\left(\left((M'^{(1)}_{sd}/M'^{(0)}_{sd}) - T_s - \sigma - T_d\right) - (T^{theo}_{sd,m}(\alpha'_1,...\alpha'_N))\right)^2}{\sigma^2\left(T_{sd}\right)}$$

mit:

$$T_{sd,m}^{theo}\left(\alpha'_1,...\alpha'_N\right) = \frac{\sum_{n=1}^{N}\alpha'_n\,G_{sm_n}G_{m_nd}\left(T_{sm_n}+T_{m_nd}\right)}{\sum_{n=1}^{N}\alpha'_n\,G_{sm_n}G_{m_nd}}$$

wobei Ts, Td und $\tau$ jeweils die Reaktionszeit der Quelle, des Detektors und die Fluoreszenzdauer des Fluorophors darstellen, $Tsm_n$ und $Tm_nd$ die jeweiligen Laufzeiten zwischen der Quelle und dem lokalisierten Fluorophor in dem Voxel $m_n$ und zwischen dem lokalisierten Fluorophor in dem Voxel $m_n$ und dem Detektor darstellen, der Koeffizient $\sigma^2$ (Tsd) einer Schätzung der Varianz der Verteilung Tsd ist, $T^{theo}_{sd}$ ($\alpha'1...\alpha'N$) dann eine Schätzung des normierten Moments der Ordnung 1 von der Funktion $M_{sd}(t)$ darstellt, die Koeffizienten $\alpha'_N$ durch die Minimierungsoperation erhalten werden, $M'^{(1)}_{sd}$ der Moment der Ordnung 1, korrigiert um die Schätzung der Beteiligung an der Laufzeit der bereits lokalisierten Fluorophore, ist.

6. Vorrichtung zum Lokalisieren von mindestens N (N > 1) Fluorophoren ($F_1$, $F_2$), wobei N eine unbekannte Zahl ist, in einem streuenden Medium, enthaltend zumindest eine gepulste Strahlungsquelle ($S_1$ - $S_4$, $L_1$ - $L_4$), die dazu geeignet ist, eine Strahlung zum Anregen dieser Fluorophore zu emittieren, und zumindest einen Detektor ($D_1$ - $D_5$), der dazu geeignet ist, ein von diesen Fluorophoren emittiertes Fluoreszenzsignal mit der gemessenen Intensität $M^0_{sd}$ zu messen, enthaltend:

- Mittel (26) zum Erstellen einer zeitlichen Verteilung $M_{sd}(t)$ des von dem Detektor empfangenen Signals für zumindest ein Quelle-Detektor-Paar,
- Mittel (26) zum Erstellen eines Gitters (M) des Volumens in Elementarzellen bzw. Voxeln, und zum Aufteilen einer aktuellen Anzahl N an Fluorophoren in dem Medium, wobei jeder Fluorophor in einem der M Voxel des Mediums gemäß einer Aufteilung m aufgeteilt ist,
- Mittel (26) zum Berechnen zumindest eines Basisparameters $\chi^N_{i,m}$ bei verschiedenen Aufteilungen m, der für zumindest eine der zeitlichen Verteilungen $M_{sd}(t)$ zumindest eine Größe, die aus zumindest einem Moment der Verteilung erhalten wird, und zumindest eine Schätzung dieser Größe kombiniert, der zumindest eine Basisparameter gibt Auskunft über den Standort von Fluorophoren;
- wobei diese Vorrichtung ferner enthält auch Mittel für, für zumindest eine Basisparameter:

* Berechnen einer Korrelation zwischen der gemessenen Intensität und den gesamten Beteiligungen an der Intensität der gesamten Fluorophore;
* Inkrementieren der aktuellen Anzahl N von Fluorophoren, wenn das Berechnen einer Korrelation nicht zufriedenstellend ist.

7. Vorrichtung nach Anspruch 6, ferner enthaltend Mittel (26) zum Ermitteln eines kombinierten Parameters $P_m^N$, der zumindest zwei Basisparameter $\chi^N_{i,m}$ kombiniert und beispielsweise gleich der Summe oder dem Produkt der Basisparameter $\chi^N_{1,m}$ und $\chi^N_{2,m}$ ist.

8. Vorrichtung nach einem der Ansprüche 6 oder 7, wobei die Mittel (26) zum Berechnen zumindest eines Parameters Mittel zum Ermitteln eines Parameters $\chi^N_{2,m}$ enthalten, der den Vergleich zwischen dem normierten Moment der Ordnung 1 der zeitlichen Verteilung, korrigiert um die Schätzung der Beteiligung an der Laufzeit der bereits lokalisierten Fluorophore, und der Modellierung dieses normierten Moments der Ordnung 1 oder zwischen dem Moment der Ordnung 0 der zeitlichen Verteilung, korrigiert um die Schätzung der Beteiligung an der Intensität der bereits lokalisierten Fluorophore, und der Modellierung dieses Moments der Ordnung 0 enthält.

9. Vorrichtung nach einem der Ansprüche 6 bis 8, wobei die Mittel (26) zum Berechnen zumindest eines Parameters Mittel enthalten zum:

- Ermitteln eines ersten Basisparameters $\chi^N_{1,m}$, der für alle Paare (Quelle, Detektor) die Summe der Differenzen zwischen dem Wert der gemessenen Intensität $M_{sd}^0$ für jedes Paar (Quelle, Detektor), korrigiert um die Schätzung der Beteiligung an der Intensität der bereits lokalisierten Fluorophore, und einer Schätzung des Moments der Ordnung null für jedes Paar (Quelle, Detektor) ist, die mit Hilfe der Greenschen Funktionen $G_{smn}$ und $G_{mnd}$ für die Quelle und den Detektor eines jeden Paares erhalten wird, wobei diese Schätzung unter der Maßgabe erfolgt, dass die N Fluorophore in den Voxeln des Mediums gemäß der Konfiguration m aufgeteilt sind, wonach die Fluorophore in den Voxeln $m_n$ aufgeteilt sind;
- Ermitteln eines zweiten Basisparameters $\chi^N_{2,m}$, der für alle Paare (Quelle, Detektor) die Summe der Differenzen für jedes Paar Quelle-Detektor zwischen der gemessenen mittleren Laufzeit Quelle-Detektor (normierter Moment der Ordnung 1 von M(t)), korrigiert um bekannte zeitliche Größen bezogen auf die Quelle, auf den Detektor und auf den Fluorophor, und korrigiert um die Schätzung der Beteiligung an der Laufzeit der bereits lokalisierten Fluorophore, und der Schätzung der korrigierten Laufzeit ist, wobei diese Schätzung unter der Maßgabe erfolgt, dass die N Fluorophore in den Voxeln des Mediums gemäß der Konfiguration m aufgeteilt sind.

**Claims**

1. A method for locating at least N (N>1) fluorophores ($F_1$, $F_2$), N being an unknown number, in a diffusing medium using at least one source ($S_1 - S_4$, $L_1-L_4$,) of pulsed radiation capable of emitting radiation to excite these fluorophores, and at least one detector ($D_1 - D_5$) capable of measuring a fluorescence signal emitted by these fluorophores comprising:

   - illumination of the medium by a radiation source,
   - detection, by at least one detector, of the signal, with a measured intensity $M^0_{sd}$, produced by the medium at the fluorescence wavelength,
   - for at least one source-detector pair, performing a temporal distribution $M_{sd}(t)$ of the signal received by the detector,
   - the diffusing medium being discretized into M voxels, considering that we have a current N number of fluorophores, each fluorophore occupying one voxel among the M voxels of the medium, according to a distribution m,
   - for different distributions m, the computing of at least one basic parameter $\chi^N_{i,m}$, which, for at least one of said temporal distributions $M_{sd}(t)$, combines at least one magnitude obtained from at least one moment of said distribution, and at least one estimation of this magnitude; the at least one basic parameter giving an information which concerns the location of the fluorophores;
   - method in which the following steps are carried out for at least one basic parameter:
   * a computing step to compute a correlation ($S_8$, $S'_{11}$) between the measured intensity and all the intensity contributions of all the fluorophores;
   - a step ($S'_1$) to increment the current N number of fluorophores if the computing of a correlation is not satisfactory.

2. The method according to claim 1, also comprising the determination of a combined parameter $P_m^N$, combining at least two basic parameters $\chi^N_{i,m}$, for example equal to the sum or to the product of two basic parameters $\chi^{N=1}_{1,m}$, and $\chi^{N=1}_{2,m}$, the combined parameters $P_m^{N=1}$ of lowest value then corresponding to the distributions of fluorophores the closest to reality.

3. The method according to one of previous claims, one of the basic parameters $\chi^N_{2,m}$ comprising a comparison between the first-order normalized moment of said temporal distribution, corrected of the time-of-flight contribution estimation of the already located fluorophores, and the modeling of this first-order normalized moment or a comparison between the zero-order moment of said temporal distribution, corrected of the intensity contribution estimation of the already located fluorophores, and the modeling of this zero-order moment.

4. The method according to one of previous claims, comprising the determination of at least one of the following basic parameters:

   - the determination of a first basic parameter $\chi^N_{1,m}$, which is the sum, for all source-detector pairs, of the differences between the value of the measured intensity $M_{sd}^0$ for each source-detector pair, corrected of the intensity contribution estimation of the already located fluorophores, and an estimation of the zero-order moment, for each source-detector pair, obtained using the Green functions $G_{smn}$ and $G_{mnd}$, for the source and the detector of each pair, this estimation being made by considering that the N fluorophores are distributed in the voxels of the medium as per configuration m, in which the fluorophores are distributed in the voxels $m_n$;
   - the determination of a second basic parameter $\chi^N_{2,m}$ which is the sum for all source-detector pairs of the differences, for each source-detector pair, between the mean measured source-detector time-of-flight (first-order normalized moment of M(t)), corrected by known temporal magnitudes relating to the source, to the detector and to the fluorophore, and corrected of the time-of-flight contribution estimation of the already located fluorophores, and the estimation of this corrected time-of-flight, this estimation being made by considering that the N fluorophores are distributed in the voxels of the medium as per configuration m.

5. The method according to claim 4, wherein the basic coefficient $\chi_{1m}^N$ is such that:

$$\chi_{1m}^N = \min_{\alpha_1,...\alpha_N} \sum_{sd} \frac{\left(M'^{(0)}_{sd} - M^{theo\ 0}_{sd,m}(\alpha_1,...\alpha_N)\right)^2}{\sigma^2\left(M^0_{sd}\right)}$$

where:

$$M^{theo\ 0}_{sd,m}(\alpha_1,...\alpha_N) = \sum_{n=1}^N \alpha_n G_{sm_n} G_{m_n d}$$

- a configuration m corresponding to N fluorophores distributed in the voxels $m_n$, $1 \leq n \leq N$,
- $G_{smn}$ (respectively $G_{mnd}$) representing the energy transfer functions between the source and the voxel $m_n$ (respectively the voxel $m_n$ and the detector d), the coefficients $\alpha_n$, being obtained by the minimization operation, as is $\chi_{1m}$;
- each element $(M'^0_{sd} - M^{theo0}_{sd,m}(\alpha_1,... \alpha_N))$ of the sum then representing the difference between:

  - the value $M'^0_{sd}$ of the measured intensity $M^0_{sd}$ for the selected source-detector pair, corrected of the intensity contribution estimation of the already located fluorophores,
  - and an estimation of the zero-order moment $M^0_{sd}$, obtained using firstly the Green functions $G_{smn}$ and $G_{mnd}$, and secondly a set of coefficients $\alpha_n$ each of which is assigned to a fluorophore and which represents the fluorescence emission intensity thereof,

and the second basic parameter $\chi_{2m}^N$ possibly being:

$$\chi_{2m}^N = \min_{\alpha'_1,...\alpha'_N} \sum_{sd} \frac{\left(\left((M'^{(1)}_{sd}/M'^{(0)}_{sd}) - T_s - \sigma - T_d\right) - (T^{theo}_{sd,m}(\alpha'_1,...\alpha'_N))\right)^2}{\sigma^2\left(T_{sd}\right)}$$

where:

$$T^{theo}_{sd,m}(\alpha'_1,...\alpha'_N) = \frac{\sum_{n=1}^N \alpha'_n G_{sm_n} G_{m_n d}\left(T_{sm_n} + T_{m_n d}\right)}{\sum_{n=1}^N \alpha'_n G_{sm_n} G_{m_n d}}$$

Ts, Td and $\tau$ respectively representing the response times of the source, of the detector and the duration of fluorescence of the fluorophore, $T_{sm_n}$ and $T_{m_n}d$ representing the respective times-of-flight between the source and the fluorophore located in voxel $m_n$, and between the fluorophore located in voxel $m_n$ and the detector, the coefficient $\sigma^2(Tsd)$ corresponding to an estimation of the distribution variance Tsd, $T^{theo}_{sd}(\alpha'1...\alpha'N)$ then representing an estimation of the first-order normalized moment of function $M_{sd}(t)$, the coefficients $\alpha'_N$ being obtained by the minimization operation, $M'^{(1)}_{sd}$ being the first-order moment corrected of the time-of-flight contribution estimation of the already located fluorophores.

6. A device for locating at least N (N>1) fluorophores ($F_1$, $F_2$), N being an unknown number, in a diffusing medium, comprising at least one pulsed radiation source ($S_1$ - $S_4$, $L_1$-$L_4$,) capable of emitting radiation to excite these fluorophores and at least one detector ($D_1$ - $D_5$) capable of measuring a fluorescence signal, with a measured intensity $M^0_{sd}$, emitted by these fluorophores, comprising:

   - means (26), for at least one source-detector pair, to perform temporal distribution $M_{sd}(t)$ of the signal received by the detector,

- means (26) to produce meshing (M) of the volume into mesh elements or voxels, and to distribute a current N number of fluorophores in the medium, each fluorophore being distributed in one of the M voxels of the medium as per a distribution m,

- means (26) to compute, for different distributions m, at least one basic parameter $\chi^N_{i,m}$, which, for at least one of said temporal distributions $M_{sd}(t)$, combines at least one magnitude obtained from at least one moment of said distribution, and at least one estimation of this magnitude; the at least one basic parameter giving an information which concerns the location of the fluorophores;

- this device further comprising means for, for at least one basic parameter:

* computing a correlation between the measured intensity and all the intensity contributions of all the fluorophores;

- incrementing the current N number of fluorophores if the computing of a correlation is not satisfactory.

7. The device according to claim 6, further comprising means (26) for determining a combined parameter $P_m^N$, combining at least two basic parameters $\chi^N_{i,m}$, for example equal to the sum or to the product of the basic parameters $\chi^N_{1,m}$, and $\chi^N_{2,m}$.

8. Device according to one of claims 6 or 7, the means (26) for computing at least one parameter comprising means for determining a parameter $\chi^N_{2,m}$ comprising the comparison between the first-order normalized moment of said temporal distribution, corrected of the time-of-flight contribution estimation of the already located fluorophores, and the modeling of this first-order normalized moment or between the zero-order moment of said temporal distribution, corrected of the intensity contribution estimation of the already located fluorophores, and the modeling of this zero-order moment.

9. The device according to one of claims 6 to 8, the means (26) to compute at least one parameter comprising means for:

- determining a first basic parameter $\chi^N_{1,m}$, which is the sum, for all source-detector pairs, of the differences between the value of the measured intensity $M_{sd}^0$ for each source-detector pair, corrected of the intensity contribution estimation of the already located fluorophores, and an estimation of the zero-order moment for each source-detector pair, obtained using the Green functions $G_{smn}$ and $G_{mnd}$, for the source and the detector of each pair, this estimation being made by considering that the N fluorophores are distributed in the voxels of the medium as per configuration m, in which the fluorophores are distributed in the voxels $m_n$;

- determining a second basic parameter $\chi^N_{2,m}$, which is the sum, for all source-detector pairs, of the differences, for each source-detector pair, between the mean measured source-detector time-of-flight (first-order normalized moment of M(t)), corrected by known temporal magnitudes relating to the source, to the detector and to the fluorophore, and corrected of the time-of-flight contribution estimation of the already located fluorophores, and the estimation of this corrected time-of-flight, this estimation being made by considering that the N fluorophores are distributed in the voxels of the medium as per configuration m.

FIG.1A

FIG.1B

FIG.1C

FIG.1D

FIG.1E

FIG.2

FIG.3A

FIG.3B

FIG.3C

0/0

Z1 = 0,2 cm    Z2 = 0,4 cm    Z3 = 0,6 cm    Z4 = 0,8 cm

Z5 = 1 cm    Z6 = 1,2 cm    Z7 = 1,4 cm    Z8 = 1,6 cm

Z9 = 1,8 cm    Z10 = 2 cm    Z11 = 2,2 cm    Z12 = 2,4 cm

Z13 = 2,6 cm    Z14 = 2,8 cm    Z15 = 3 cm

FIG.4A

$$x\ 10^{-10}$$

FIG.4B

FIG. 4C

Z1 = 0,2 cm    Z2 = 0,4 cm    Z3 = 0,6 cm    Z4 = 0,8 cm

Z5 = 1 cm    Z6 = 1,2 cm    Z7 = 1,4 cm    Z8 = 1,6 cm

Z9 = 1,8 cm    Z10 = 2 cm    Z11 = 2,2 cm    Z12 = 2,4 cm

Z13 = 2,6 cm    Z14 = 2,8 cm    Z15 = 3 cm

# FIG. 5A

2    4    6    8    10    12    14    16    18

x 10$^8$

Z1 = 0,2 cm    Z2 = 0,4 cm    Z3 = 0,6 cm    Z4 = 0,8 cm

Z5 = 1 cm    Z6 = 1,2 cm    Z7 = 1,4 cm    Z8 = 1,6 cm

Z9 = 1,8 cm    Z10 = 2 cm    Z11 = 2,2 cm    Z12 = 2,4 cm

Z13 = 2,6 cm    Z14 = 2,8 cm    Z15 = 3 cm

# FIG. 5B

0    1    2    3    4    5    6    7    8    9

x 10$^8$

FIG. 6

FIG.7

$S_0$ : X = 0

$S_1$ : $X_{m,n} = 0$

$S_2$ : Calcul de $M_{sd}^{(0)}$ et/ou $M_{sd}'^{(1)}$

$S_3$ : $M_{sd}^{(0)} > 0$   $M_{sd}'^{(1)} > 0$

$S_4$ : Calcul de $\chi i$, m, Pm

$S_5$ : Xmn = paramètre pertinent ($\chi im$ ou Pm)

$S_6$ : Ajustement de X:
- détermination des $\alpha i$
- convergence ?

$S_7$ : n = N ?   N

n = n+1

$S_8$ : Corrélation OK ?   N

$S_9$ : FIN

## FIG.8

$$N = 0 \quad S'_0$$

$$N = N+1 \quad S'_1$$

$$X_{mn} = 0 \quad S'_2$$

$$N_{iter} = 0 \quad S'_3$$

$$N_{iter} = N_{iter}+1 \quad S'_4$$

Calcul de $M_{sd}^{(0)}$
et/ou
$M'^{(1)}_{sd}$   $S'_5$

$M_{sd}^{(0)} > 0$

$M'^{(1)}_{sd} > 0$   $S'_6$

Calcul de $\chi i$, m, Pm   $S'_7$

Xmn = paramètre pertinent
($\chi im$ ou Pm)   $S'_8$

n = n+1

Ajustement de X:
- détermination des $\alpha i$
- convergence   $S'_9$

$S'_{10}$  n = N ?   N

O

$S'_{11}$  Corrélation OK?   O

- N
- X est déterminée   $S'_{12}$

N

N   $N_{iter}$ max ?

FIN   $S'_{13}$

$S'_{14}$

O

FIG.9A

FIG.9B

FIG.9C

## FIG.10A

## FIG.10B

## FIG.10C

## FIG.10D

## FIG.10E

## FIG.10F

## FIG.10G

## FIG.10H

FIG.11A

FIG.11B

Z1 = 0,2 cm     Z2 = 0,4 cm     Z3 = 0,6 cm     Z4 = 0,8 cm

Z5 = 1 cm       Z6 = 1,2 cm     Z7 = 1,4 cm     Z8 = 1,6 cm

Z9 = 1,8 cm     Z10 = 2 cm      Z11 = 2,2 cm    Z12 = 2,4 cm

Z13 = 2,6 cm    Z14 = 2,8 cm    Z15 = 3 cm

0    0,1   0,2   0,3   0,4   0,5   0,6   0,7   0,8   0,9   1

# FIG.11C

# FIG.12A

FIG.12B

FIG.12C

**EP 2 302 362 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

*   EP 1884765 A
*   WO 200632151 A

**Littérature non-brevet citée dans la description**

*   **S.R. ARRIDGE.** Optical tomography in medical imaging. *Inverse Problems,* Avril 1999, vol. 15 (2), R41-R93